# EUROPEAN PATENT APPLICATION

(11) **EP 4 657 055 A1**
(43) Date of publication of application: **03.12.2025**
(21) Application number: 23922248.2
(22) Date of filing: 18.07.2023
(51) Int. Cl.: G01N 27/26, G01N 27/416, G01N 33/52, G01N 27/06, G01R 27/22, G01R 35/00

(54) **METABOLIC INDEX CONCENTRATION MEASUREMENT METHOD AND SYSTEM AND ELECTROCHEMICAL MEASUREMENT SYSTEM**

(30) Priority: 15.02.2023 CN 202310113959
(71) Applicant: COFOE MEDICAL TECHNOLOGY CO., LTD., Changsha, Hunan 410007 (CN)
(72) Inventor: QUAN, Changyun, Changsha, Hunan 410007 (CN); QIU, Jing, Changsha, Hunan 410007 (CN); LIU, Lixia, Changsha, Hunan 410007 (CN)
(74) Representative: Alpspitz IP
(86) International application number: PCT/CN2023/107916
(87) International publication number: WO 2024/169125

(57) **Abstract**

A metabolic index concentration measurement method and system and an electrochemical measurement system. The impedance value of a blood sample is compared with a standard impedance value in a preset standard impedance library, a hematocrit correction range is divided according to a certain interval, the interval in which a hematocrit is located corresponding to the blood sample is determined according to the comparison result, and a metabolic index concentration equation is determined under corresponding hematocrit conditions, and then obtained metabolic indexes are weighted and averaged. The measurement accuracy of low, medium, and high concentration ranges of different measurement indexes is improved.

## Description

### Field of the Invention

The present invention relates to the technical field of electroanalytical measurement, in particular to a metabolic index concentration measurement method and system and an electrochemical measurement system.

### Background of the Invention

Diabetes is one of the major metabolic diseases that endanger human health, mainly manifested as abnormally high levels of glucose in blood and urine. Meanwhile, with the continuous improvement of living standards and gradual changes in lifestyle, the number of people with high uric acid levels has been increasing steadily. Like hyperglycemia, hyperuricemia is a metabolic disorder, and the two influence each other. Research indicates that for every 60 µmol/L increase in blood uric acid levels, the risk of developing diabetes rises by 18%. Therefore, measuring blood glucose and uric acid levels is of critical importance for the prevention and diagnosis of chronic diseases.

Currently, single-parameter test strips for blood glucose or uric acid dominate the market, most of which contain only one reaction zone and are thus limited to individual biomarker measurement. To achieve multi-component measurement, repeated sampling is required, which not only creates user inconvenience but also leads to significant test strip consumption.

In conclusion, developing an electrochemical measurement system for simultaneous blood glucose and uric acid measurement with hematocrit (HCT) correction capability is of great significance for improving the accuracy of measurement results and facilitating real-time user monitoring.

### Summary of the Invention

In view of the shortcomings of the prior art, the technical problem to be solved by the present invention is to provide a metabolic index concentration measurement method and system and an electrochemical measurement system, to improve the accuracy of measurement results.

To solve the above technical problems, the technical solution adopted by the present invention is as follows: A metabolic index concentration measurement method includes the following steps:
S1, measuring an impedance value and a metabolic index current value of a blood sample; comparing the measured impedance value with a preset standard impedance library, and determining a hematocrit range corresponding to the blood sample based on the comparison result, where the preset standard impedance library include different hematocrit levels and standard impedance values corresponding to the hematocrit levels;
S2, searching for metabolic index concentration equations corresponding to the hematocrit range;
S3, substituting the metabolic index current value into the metabolic index concentration equations Y1 and Y2 to obtain metabolic index concentrations C1 and C2 respectively; and
S4, substituting the metabolic index concentrations C1 and C2 into the following formula to calculate a metabolic index concentration value Fn: Fn={(a-a1)C2/(a2-a1)}+{(a2-a)C1/(a2-a1)};
where Y1 and Y2 represent the metabolic index concentration equations corresponding to an upper limit value and a lower limit value of the hematocrit range respectively, a represents the measured impedance value of the blood sample, a1 represents that the hematocrit value is a standard impedance value corresponding to the lower limit value of the hematocrit range, and a2 represents that the hematocrit value is a standard impedance value corresponding to the upper limit value of the hematocrit range.

For example, the metabolic index is blood glucose and/or uric acid: in step S3, a blood glucose current value and/or a uric acid current value of the blood sample are measured, and the blood glucose current value is substituted into blood glucose concentration equations Y1 and Y2 to obtain blood glucose concentrations C1 and C2 respectively; and/or
the uric acid current value is substituted into uric acid concentration equations J1 and J2 to obtain uric acid concentrations D1 and D2 respectively;
in step S4, the blood glucose concentrations C1 and C2 are substituted into the following formula to calculate a blood glucose concentration value - metabolic index concentration value C (at this time, Fn=C): C={(a-a1)C2/(a2-a1)}+{(a2-a)C1/(a2-a1)}; and/or the uric acid concentrations D1 and D2 are substituted into the following formula to calculate a uric acid concentration value D (at this time, Fn=D): D={(a-a1)D2/(a2-a1)}+{(a2-a)D1/(a2-a1)};
where J1 and J2 represent the uric acid concentration equations corresponding to the upper limit value and lower limit value of the hematocrit range respectively.

In the present invention, the impedance value of the blood sample is compared with the standard impedance values in the preset standard impedance library, the hematocrit correction range is divided at certain intervals, the range of hematocrit corresponding to the blood sample is determined based on the comparison results, the metabolic index concentration equations are determined under the corresponding hematocrit conditions, and then weighted averaging is performed on the obtained metabolic indexes, thereby improving the accuracy of measurement at low, medium, and high concentrations of different measurement indexes.

The present invention can simultaneously implement the measure of metabolic index concentrations (blood glucose concentration and uric acid concentration) and HCT correction, and the measurement results of different metabolic index concentrations, such as blood glucose concentration and uric acid concentration, do not interfere with one another, thereby improving the accuracy of measurement results.

It is not difficult to understand that the solution of the present invention is also applicable to the concentration measurement of common electrochemical measurement indexes, such as cholesterol, triglycerides, hemoglobin, β-hydroxybutyric acid, and creatinine.

Further, in step S1, a process of obtaining standard impedance values in the standard impedance library includes:
1) obtaining a plurality of original blood samples;
2) for each of the original blood samples, preparing M blood samples with hematocrit levels of N1%, N2%... NM%, where N1, N2... NM are all positive integers, and N1 < N2 <... < NM; and
3) measuring the impedance values of each blood sample at the corresponding hematocrit levels respectively, and determining an average value of all impedance values at the same hematocrit level as the standard impedance value corresponding to that hematocrit level.

In the present invention, the standard impedance library including different hematocrit levels and standard impedance values corresponding to the hematocrit levels is constructed, and the standard impedance value corresponding to each hematocrit level is set as an average value of all impedance values at that hematocrit level, thereby improving the accuracy of the standard impedance values, ensuring that the measured impedance value can be matched to the accurate hematocrit range, and further improving the accuracy of measurement.

Further, in extreme cases, in step S1, if it is determined based on the comparison result that the hematocrit value of the blood sample may be equal to the lower limit value or upper limit value of the hematocrit range, the lower limit value and upper limit value of the hematocrit range corresponding to the blood sample are set to be equal to the hematocrit value of the blood sample determined based on the comparison result.

Further, in extreme cases, in step S1, if it is determined based on the comparison result that the difference between the hematocrit value of the blood sample and a minimum value of lower limit values of all hematocrit ranges is not greater than a second set threshold (for example, not more than 5%), the lower limit value and upper limit value of the hematocrit range corresponding to the blood sample are both set as the minimum value; and if it is determined based on the comparison result that the difference between the hematocrit value of the blood sample and a maximum value of upper limit values of all hematocrit ranges is not greater than the second set threshold, the lower limit value and upper limit value of the hematocrit range corresponding to the blood sample are both set as the maximum value.

For example, N1%, N2%... NM% correspond to 10%, 20%... 70% respectively. When the hematocrit value of the blood sample determined based on the comparison result is 9.5%, the lower limit value and upper limit value of the hematocrit range corresponding to the blood sample are both set at 10%. When the hematocrit value of the blood sample determined based on the comparison result is 70.5%, the lower limit value and upper limit value of the hematocrit range corresponding to the blood sample are both set at 70%. In the above two extreme cases, step S4 is replaced with:
the metabolic index concentration value Fn=C1 or C2.

Further, in step S2, a process of establishing metabolic index concentration equations includes: obtaining an original blood sample, preparing the original blood sample into M blood samples with different hematocrit levels, dividing the blood sample corresponding to each hematocrit level into M equal parts, preparing the M equal parts of blood samples into blood samples with different metabolic index concentrations, and calibrating the blood samples with different hematocrit levels and different metabolic index concentrations to establish a metabolic index concentration - current value relation curve, thereby obtaining the metabolic index concentration equations corresponding to different hematocrit levels.

When the metabolic indexes are blood glucose and uric acid, the process of establishing metabolic index concentration equations includes:
obtaining an original blood sample, preparing the original blood sample into M blood samples with different hematocrit levels, dividing the blood sample corresponding to each hematocrit level into M equal parts, preparing the M equal parts of blood samples into blood samples with different glucose/uric acid concentrations, and calibrating the blood samples with different hematocrit levels and different glucose/uric acid concentrations to establish a blood glucose concentration - blood glucose current value relation curve or a uric acid concentration - uric acid current value relation curve, thereby obtaining blood glucose concentration equations or uric acid concentration equations corresponding to different hematocrit levels.

The present invention establishes the metabolic index concentration equations corresponding to different hematocrit levels (such as blood glucose concentration equations, uric acid concentration equations, or cholesterol concentration equations), facilitating the search for the corresponding blood glucose concentration equations or uric acid concentration equations within the corresponding hematocrit range, accelerating calculation, and improving calculation accuracy.

Further, in the present invention, an expression of the blood glucose concentration - blood glucose current value relation curve is: Yᵢ=b*xᵢ²+c*xᵢ+d, where xᵢ represents a blood glucose current value, Yᵢ represents a blood glucose concentration value, and b, c, and d all represent constants; and an expression of the uric acid concentration - uric acid current value relation curve is: Jᵢ=e*yᵢ+f, where e and f all represent constants, yᵢ represents a uric acid current value, and Jᵢ represents a uric acid concentration value.

Based on the same inventive concept, the present invention further provides a metabolic index concentration measurement system, including:
an HCT measurement module, configured to measure an impedance value, compare the measured impedance value with a preset standard impedance library, determine a hematocrit range corresponding to the blood sample based on the comparison result, and search for metabolic index concentration equations corresponding to the hematocrit range;
a metabolic index current value measurement module, configured to measure a metabolic index current value of the blood sample; and
a calculation unit, configured to substitute the metabolic index current value obtained by the metabolic index current value measurement module into metabolic index concentration equations Y1 and Y2 respectively to obtain metabolic index concentrations C1 and C2, and substitute the metabolic index concentrations C1 and C2 into the following formula to calculate a metabolic index concentration value Fn: Fn={(a-a1)C2/(a2-a1)}+{(a2-a)C1/(a2-a1)};
where Y1 and Y2 represent blood glucose concentration equations corresponding to an upper limit value and a lower limit value of the hematocrit range respectively, a represents the measured impedance value of the blood sample, a1 represents that the hematocrit value is a standard impedance value corresponding to the lower limit value of the hematocrit range, and a2 represents that the hematocrit value is a standard impedance value corresponding to the upper limit value of the hematocrit range.

In the present invention, to facilitate data comparison and search, the standard impedance library and the metabolic index concentration equations corresponding to each hematocrit range are all stored in a data unit.

In the present invention, to facilitate users' timely monitoring of relevant metabolic indexes, the aforementioned metabolic index concentration measurement system further includes:
a display unit, configured to display the metabolic index concentration value Fn.

In the present invention, the interval between two adjacent hematocrit values can be set at 10%, that is, the span of the hematocrit range is 10%, thereby improving calculation speed on the premise of ensuring calculation accuracy.

Based on the same inventive concept, the present invention further provides another metabolic index concentration measurement method, including the following steps:
A. applying a plurality of different frequencies to an electrode module of an electrochemical test strip;
B. obtaining concentration values of a metabolic index of a blood sample at different frequencies; and
C. performing weighted averaging on the concentration values of the metabolic index of the same blood sample at the different frequencies to obtain a corrected concentration value of the metabolic index corresponding to the blood sample;
where the concentration value of the metabolic index of the blood sample at a certain frequency is obtained by the measurement method in the above embodiments.

In the present invention, sine waves of different frequencies are applied to the electrode module of the electrochemical test strip, the concentration values of the to-be-measured index (metabolic index) are obtained by using the impedance values and current values of the blood sample at different frequencies, and then weighted averaging is performed on the concentration values of the to-be-measured index at different frequencies to obtain the final corrected concentration value of the to-be-measured index of the blood sample. In the present invention, the plurality of frequencies is used to measure the HCT impedance of the same blood sample, the plurality of concentration values of the to-be-measured index of the same sample at different frequencies can be obtained through conversion, and then weighted averaging is performed to obtain the final concentration of the to-be-measured index of the sample, thereby reducing the probability of impedance outliers and improving the accuracy of different hematocrit correction. On the other hand, compared with a single-frequency measurement method, multi-frequency collection can expand the hematocrit range of the blood sample, and its hematocrit correction range can be 0-70%, which can be directly measured by using a plasma sample. The present invention employs different frequencies for impedance collection and HCT correction, thereby improving the accuracy of impedance collection for different populations, making the HCT correction method applicable to different populations, and greatly improving the accuracy of corrected concentration results of to-be-measured indexes.

To further improve the accuracy of corrected results, after step A and before step B, the method further includes:
comparing the impedance values at different frequencies with a standard impedance library of different frequencies to determine whether the hematocrit ranges of the blood sample at different frequencies meet set requirements, and if so, performing step B; otherwise, issuing an alarm instruction.

In the present invention, the set requirements include:
if the number of frequencies n is 2, the hematocrit ranges selected at the two frequencies are located within an adjacent or same hematocrit range;
if the number of frequencies n is 3, at least two of the hematocrit ranges selected at the three frequencies are located within a same hematocrit range, and the other one is located within an adjacent hematocrit range; and
if the number of frequencies n is greater than or equal to 4, at least n-2 of the hematocrit ranges selected at n different frequencies are located within a same hematocrit range, and the remaining ones are located within adjacent hematocrit ranges.

In the present invention, if the set requirements are met, subsequent measurements continue; otherwise, the alarm instruction will be issued. This determination process can further ensure the accuracy of the corrected results.

Corresponding to the aforementioned measurement method, the present invention further provides a metabolic index concentration measurement system, including:
a frequency control unit, configured to apply a plurality of different frequencies to an electrode module of an electrochemical test strip;
a metabolic index concentration value obtaining unit, configured to obtain concentration values of a metabolic index of a blood sample at different frequencies; and
a corrected concentration value calculation unit, configured to perform weighted averaging on the concentration values of the metabolic index of the same blood sample at the different frequencies to obtain a corrected concentration value of the metabolic index corresponding to the blood sample;
where the metabolic index concentration value obtaining unit is the aforementioned metabolic index concentration measurement system.

The above system of the present invention further includes: a determination unit, configured to compare the impedance values at different frequencies with a standard impedance library of different frequencies to determine whether the hematocrit ranges of the blood sample at different frequencies meet set requirements, and if so, send a working instruction to the metabolic index concentration value obtaining unit, otherwise, issue an alarm instruction.

Based on the same inventive concept, the present invention further provides an electrochemical measurement system, including:
an electrochemical test strip; and
a linker, configured to transmit corresponding reaction signals generated by a sample on the electrochemical test strip to a measurement unit;
where the measurement unit is the aforementioned metabolic index concentration measurement system.

Compared with the prior art, the beneficial effects of the present invention are as follows:
1. The measurement method of the present invention divides a hematocrit correction range at certain intervals and determines a group of metabolic index concentration equations under corresponding hematocrit conditions, which can improve the accuracy of measurement at low, medium, and high concentrations of different measurement indexes.
2. The present invention can calibrate the measurement results of different HCT blood samples, and can control the relative deviations of the measurement results within ±8% from true values, thereby improving the accuracy of measurement.
3. The present invention can simultaneously implement the measurement and HCT correction of two indexes, and their measurement results do not incur relevant interference, where the CV (coefficient of variation) of blood glucose concentration can be controlled within 4.0%, and the CV of uric acid concentration can be controlled within 4.5%.
4. The present invention improves the accuracy of different hematocrit correction. The multi-frequency collection can expand the hematocrit range of a blood sample, and its hematocrit correction range can be 0-70%. The present invention employs different frequencies for impedance collection and HCT correction, thereby improving the accuracy of impedance collection for different populations, making the HCT correction method applicable to different populations, and greatly improving the accuracy of corrected concentration results of to-be-measured indexes. The present invention can calibrate the measurement results of different HCT blood samples, and can control the relative deviations of the measurement results within ±6.5% from true values.

### Brief Description of the Drawings

FIG. 1 is a flowchart of a measurement method in Example 1 of the present invention;
FIG. 2 is a diagram of a linear blood glucose equation in Example 1 of the present invention;
FIG. 3 is a diagram of a uric acid concentration equation in Example 1 of the present invention;
FIG. 4 is a structure diagram of a measurement system in Example 2 of the present invention;
FIG. 5 is a measurement flowchart of the measurement system in Example 2 of the present invention;
FIG. 6 is a specific measurement process diagram of the measurement system in Example 2 of the present invention;
FIG. 7 is a measurement principle diagram in Example 3 of the present invention;
FIG. 8 shows a process of calculating concentration values within different hematocrit ranges in Example 3 of the present invention;
FIG. 9 is a measurement flowchart in Example 3 of the present invention;
FIG. 10 is a diagram of a cholesterol standard equation in Example 3 of the present invention; and
FIG. 11 shows specific measurement steps in Example 4 of the present invention.

### Detailed Description of the Embodiments

In order to make the objectives, technical solutions, and advantages of the embodiments of the present invention clearer, the following will provide a clear and complete explanation of the technical solutions in the embodiments of the present invention with reference to the accompanying drawings therein. Obviously, the described embodiments are some of the embodiments of the present invention, not all of them. All other embodiments obtained by those of ordinary skill in the art based on the embodiments of the present invention without any creative efforts shall fall within the scope of protection of the present invention.

Herein, the term "include", "comprise", and other similar terms are intended to represent logical interrelationships, and cannot be regarded as representing spatial relationships. For example, "A includes B" represents that B logically belongs to A, and does not represent that B is spatially located inside A. In addition, the meanings of the terms "include", "comprise", and other similar terms should be considered open rather than closed. For example, "A includes B" represents that B belongs to A, but B may not necessarily constitute all of A, and A may further include other elements such as C, D, and E.

### Example 1

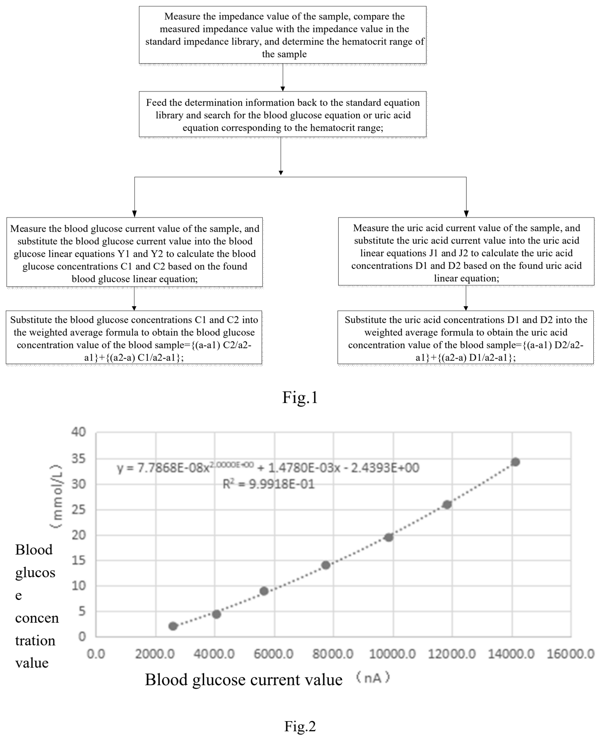

This embodiment provides a blood glucose and uric acid measurement method. As shown in FIG. 1, the measurement method includes the following steps:
Step 1: Upon blood absorption by a test strip, measure an impedance value of a sample, and compare the measured impedance value with impedance values in a standard impedance library to determine a hematocrit range of the sample;
Step 2: Feed determination information from step 1 back to a standard equation library, and search for blood glucose equations or uric acid equations corresponding to the hematocrit range;
Step 3: Measure a blood glucose current value and a uric acid current value of the sample; based on the blood glucose equations or uric acid equations found in step 2, substitute the blood glucose current value into the linear blood glucose equations Y1 and Y2 respectively to calculate blood glucose concentrations C1 and C2, and substitute the uric acid current value into the uric acid concentration equations J1 and J2 respectively to calculate uric acid concentrations D1 and D2;
Step 4: based on the measurement results in step 3, substitute the blood glucose concentrations C1 and C2 into a weighted average formula to obtain a blood glucose concentration value of the blood sample C={(a-a1)C2/(a2-a1)}+{(a2-a)C1/(a2-a1)}; substitute the uric acid concentrations D1 and D2 into a weighted average formula to obtain a uric acid concentration value of the blood sample D={(a-a1)D2/(a2-a1)}+{(a2-a)D1/(a2-a1)},
where a represents the measured impedance value of the to-be-measured sample; a1 represents that HCT is a standard impedance value within a lower limit value of the hematocrit range (for example, 10%); a2 represents that HCT is a standard impedance value within an upper limit value of the hematocrit range (for example, 20%); C1 represents a blood glucose concentration value calculated by substituting the blood glucose current value of the to-be-measured blood into the blood glucose equation with HCT of 10%; C2 represents a blood glucose concentration value calculated by substituting the blood glucose current value of the to-be-measured blood into the blood glucose equation with HCT of 20%; D1 represents a uric acid concentration value calculated by substituting the uric acid current value of the to-be-measured blood into the uric acid equation with HCT of 10%; and D2 represents a uric acid concentration value calculated by substituting the uric acid current value of the to-be-measured blood into the uric acid equation with HCT of 20%.

In this embodiment, the standard impedance library is established as follows: 3 male blood samples and 3 female blood samples are randomly drawn and prepared into 10%, 20%, 30%, 40%, 50%, 60%, and 70% blood samples respectively, the impedance value of each sample is measured, and then the impedance values at corresponding hematocrit levels are averaged to obtain the following standard impedance library (Table 1):

**Table 1 Standard impedance library**

| Serial number | Hematocrit of the blood sample | Impedance value |
|---|---|---|
| 1 | 10% | 6000 |
| 2 | 20% | 6700 |
| 3 | 30% | 7650 |
| 4 | 40% | 9200 |
| 5 | 50% | 10700 |
| 6 | 60% | 13000 |
| 7 | 70% | 17300 |

In step 1 above, upon blood absorption by the test strip, the impedance value of the blood sample is measured, and the measured impedance value is compared with the impedance values in the standard impedance library. For example, if the measured impedance value is 6600, it can be known that the hematocrit of the blood sample is within a range of 10% to 20%, that is, the lower limit value of the range is 10% and the upper limit value is 20%.

In this embodiment, the standard equation library is established as follows: a blood sample is randomly drawn and prepared into 7 blood samples with different hematocrit levels (10%, 20%, 30%, 40%, 50%, 60%, 70%), and the blood sample at each hematocrit level is further divided into 7 equal parts and prepared into blood samples with different glucose concentrations; for the blood samples with different hematocrit levels and different glucose concentrations, glucose and uric acid calibration is performed (establishing a blood glucose concentration - blood glucose current value relation curve and a uric acid concentration - uric acid current value relation curve) to obtain a group of linear blood glucose equations (Y1, Y2, Y3, Y4, Y5, Y6, Y7) and a group of uric acid concentration equations (J1, J2, J3, J4, J5, J6, J7). For example, a calibration process for a 40% hematocrit blood sample is as follows:

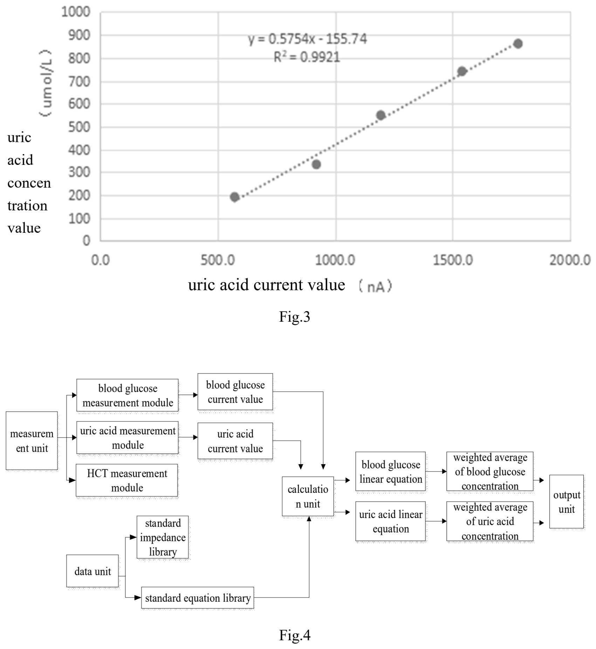

A blood sample is drawn and prepared into a 40% blood sample, the blood sample is divided into 2 equal parts, which are then prepared into 7 blood samples with different glucose concentrations and 5 blood samples with different uric acid concentrations respectively for calibration, and data is collected. The blood glucose measurement calibration is shown in Table 2, and the linear blood glucose equation is shown in FIG. 2. The uric acid measurement calibration is shown in Table 3, and the uric acid concentration equation is shown in FIG. 3. By analogy, a group of linear blood glucose equations and a group of uric acid concentration equations of blood samples with different hematocrit levels can be obtained. The standard equation library is shown in Table 4.

**Table 2 Blood glucose measurement calibration (hematocrit 40%)**

| Blood sample | 1 | 2 | 3 | 4 | 5 | 6 | 7 |
|---|---|---|---|---|---|---|---|
| Bioche mical value (mmol/ L) | 2.05 | 4.37 | 8.94 | 14 | 19.68 | 26.08 | 34.19 |
| | 2.07 | 4.38 | 8.89 | 13.94 | 19.21 | 25.63 | 34.18 |
| Bioche mical mean | 2.06 | 4.375 | 8.915 | 13.97 | 19.445 | 25.855 | 34.185 |

| Serial number | Current value (nA) | | | | | | |
|---|---|---|---|---|---|---|---|
| 1 | 3018 | 3971 | 5762 | 8120 | 9537 | 11950 | 13579 |
| 2 | 3216 | 3968 | 5661 | 8009 | 9649 | 11844 | 13921 |
| 3 | 3019 | 4007 | 5475 | 7966 | 9526 | 11419 | 14078 |
| 4 | 3304 | 4194 | 5520 | 7748 | 9666 | 11105 | 13527 |
| 5 | 2962 | 4284 | 5448 | 7535 | 9517 | 11293 | 13848 |
| 6 | 2931 | 3965 | 5516 | 7579 | 9629 | 11733 | 14028 |
| 7 | 2400 | 4221 | 5418 | 7390 | 9203 | 11627 | 14138 |
| 8 | 2407 | 4335 | 5793 | 7489 | 9930 | 11695 | 14496 |
| 9 | 2319 | 4000 | 5897 | 7485 | 9710 | 11911 | 14089 |
| 10 | 2359 | 3816 | 5839 | 7511 | 9488 | 12293 | 14086 |
| 11 | 2524 | 4196 | 5786 | 8046 | 10317 | 12124 | 14103 |
| 12 | 2424 | 3985 | 5446 | 7710 | 10191 | 12132 | 13965 |
| 13 | 2387 | 3864 | 5803 | 7953 | 10066 | 12305 | 14221 |
| 14 | 2326 | 3875 | 5681 | 7703 | 9906 | 12002 | 14349 |
| 15 | 2410 | 4181 | 5722 | 8106 | 10142 | 11346 | 14320 |
| 16 | 2554 | 4132 | 5937 | 7819 | 10145 | 11658 | 14221 |
| 17 | 2351 | 4165 | 5711 | 7869 | 10660 | 12108 | 14450 |
| 18 | 2448 | 4160 | 5685 | 7784 | 10165 | 12357 | 14478 |
| 19 | 2538 | 4138 | 5709 | 7654 | 9977 | 12037 | 14446 |
| 20 | 2313 | 4064 | 5739 | 7676 | 10185 | 11928 | 14556 |
| AV | 2610.5 | 4076.1 | 5677.4 | 7757.6 | 9880.5 | 11843.4 | 14145.0 |
| SD | 328.05 | 145.02 | 156.16 | 222.92 | 357.93 | 354.51 | 287.47 |
| CV | 12.57% | 3.56% | 2.75% | 2.87% | 3.62% | 2.99% | 2.03% |

**Table 3 Uric acid measurement calibration (hematocrit 40%)**

| Blood sample | 1 | 2 | 3 | 4 | 5 |
|---|---|---|---|---|---|
| Biochemi cal value (mmol/L) | 2.05 | 4.37 | 8.94 | 14 | 19.68 |
| | 2.07 | 4.38 | 8.89 | 13.94 | 19.21 |
| Biochemi cal mean | 2.06 | 4.375 | 8.915 | 13.97 | 19.445 |

| Serial number | Current value (nA) | | | | |
|---|---|---|---|---|---|
| 1 | 3018 | 3971 | 5762 | 8120 | 9537 |
| 2 | 3216 | 3968 | 5661 | 8009 | 9649 |
| 3 | 3019 | 4007 | 5475 | 7966 | 9526 |
| 4 | 3304 | 4194 | 5520 | 7748 | 9666 |
| 5 | 2962 | 4284 | 5448 | 7535 | 9517 |
| 6 | 2931 | 3965 | 5516 | 7579 | 9629 |
| 7 | 2400 | 4221 | 5418 | 7390 | 9203 |
| 8 | 2407 | 4335 | 5793 | 7489 | 9930 |
| 9 | 2319 | 4000 | 5897 | 7485 | 9710 |
| 10 | 2359 | 3816 | 5839 | 7511 | 9488 |
| 11 | 2524 | 4196 | 5786 | 8046 | 10317 |
| 12 | 2424 | 3985 | 5446 | 7710 | 10191 |
| 13 | 2387 | 3864 | 5803 | 7953 | 10066 |
| 14 | 2326 | 3875 | 5681 | 7703 | 9906 |
| 15 | 2410 | 4181 | 5722 | 8106 | 10142 |
| 16 | 2554 | 4132 | 5937 | 7819 | 10145 |
| 17 | 2351 | 4165 | 5711 | 7869 | 10660 |
| 18 | 2448 | 4160 | 5685 | 7784 | 10165 |
| 19 | 2538 | 4138 | 5709 | 7654 | 9977 |
| 20 | 2313 | 4064 | 5739 | 7676 | 10185 |
| AV | 2610.5 | 4076.1 | 5677.4 | 7757.6 | 9880.5 |
| SD | 328.05 | 145.02 | 156.16 | 222.92 | 357.93 |
| CV | 12.57% | 3.56% | 2.75% | 2.87% | 3.62% |

**Table 4 Standard equation library**

| | | | |
|---|---|---|---|
| 1 | 10% | Y1 | J1 |
| 2 | 20% | Y2 | J2 |
| 3 | 30% | Y3 | J3 |
| 4 | 40% | Y4 | J4 |
| 5 | 50% | Y5 | J5 |
| 6 | 60% | Y6 | J6 |
| 7 | 70% | Y7 | J7 |

The blood glucose concentration equations Y1 and Y2 constructed in this embodiment are quadratic equations with one variable, their format is shown in FIG. 2, and their expression is Yᵢ=b*xᵢ²+c*xᵢ +d, where xᵢ represents a blood glucose current value, Yᵢ represents a blood glucose concentration value, and b, c, and d are all constant terms.

The uric acid concentration equations J1 and J2 constructed in this embodiment are linear equations with one variable, their format is shown in FIG. 3, and their expression is Jᵢ=e*yᵢ+f, where yᵢ represents a uric acid current value, Jᵢ represents a uric acid concentration value, and e and f are constant terms.

### Example 2

This embodiment provides a blood glucose and uric acid measurement system. As shown in FIG. 4, the measurement system includes a measurement unit, a data unit, a calculation unit, and an output unit, where the measurement unit includes a blood glucose measurement module, a uric acid measurement module, and an HCT measurement module; the data unit is configured to store a standard impedance library and a standard equation library; the calculation unit is configured to calculate a blood glucose concentration and an uric acid concentration, and finally, the blood glucose concentration and the uric acid concentration are displayed on a screen through the output unit.

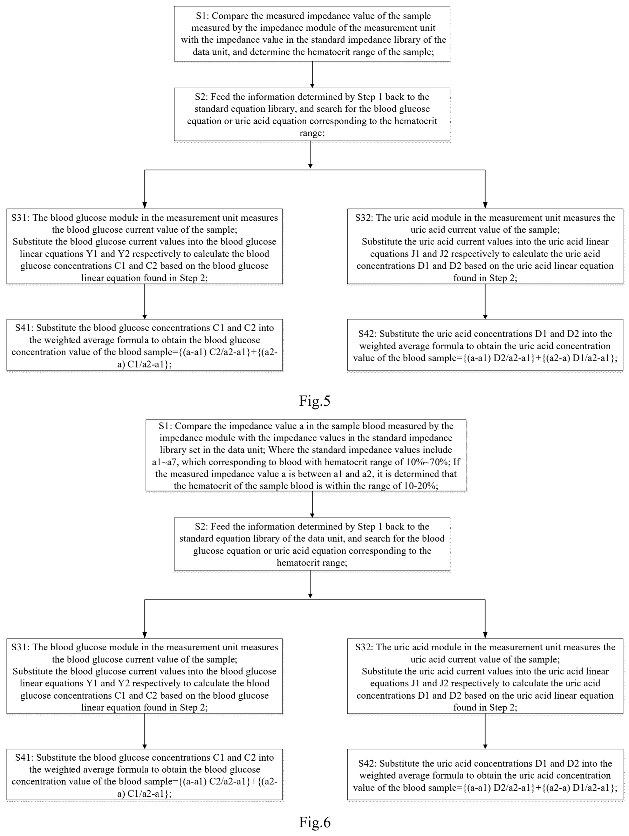

In this embodiment, after blood is automatically drawn into a test strip, firstly, each module of the measurement unit is activated for measurement, where the HCT measurement module measures an impedance value, the blood glucose measurement module measures a blood glucose current value, and the uric acid measurement module measures a uric acid current value; the measurement conditions for the HCT measurement module include an amplitude of 0.1 to 0.3 V, a frequency of 1 to 10 KHz, and a measurement time of 0.1 to 2 s, and preferably, an amplitude of 0.2 V, a frequency of 5 KHz, and a measurement time of 0.1 s; the measurement conditions for the blood glucose measurement module include a voltage of 0.2 to 0.3 V and a time of 5 to 8 s, and preferably, a voltage of 0.3 V and a time of 5 s; and the measurement conditions for the uric acid measurement module include a voltage of 0.35 to 0.45 V and a pressure time of 5 to 20 s, and preferably, a voltage of 0.4 V and a pressure time of 10 s. Then, the data unit is activated, the impedance value of a measured sample is compared with the standard impedance library in the data unit to determine a hematocrit range of the sample, the standard equation library is searched through the hematocrit range for corresponding blood glucose equations and uric acid equations, the calculation unit substitutes the blood glucose current value and uric acid current value of the corresponding sample into the corresponding matching equations respectively to obtain blood glucose concentrations or uric acid concentrations, which are then subjected to weighted averaging to obtain a final result, and the final result is displayed on the screen through the output unit (display unit). Specific measurement processes are shown in FIG. 5 and FIG. 6.

The calculation process/working process of each unit in this embodiment is identical to Example 1, and will not be elaborated here.

### Example 3

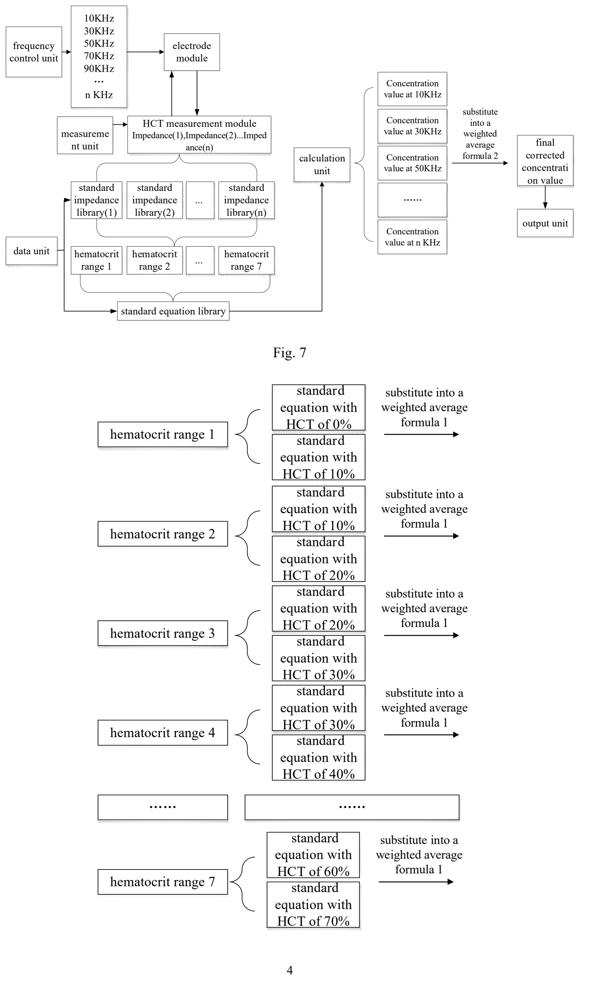

As shown in FIG. 7 to FIG. 9, this embodiment provides another metabolic index concentration measurement method, including the following steps:
A. A plurality of different frequencies are applied to an electrode module of an electrochemical test strip;

In this embodiment, sine waves of different frequencies are applied to the electrode module of the electrochemical test strip, and the impedance values of a blood sample in the electrode module at different frequencies are collected. Upon blood absorption by the test strip, different frequencies are applied to the electrode module, the impedance values of the blood sample in the electrode module are measured at different frequencies, and multiple groups of impedance values of the blood sample at different frequencies are collected.

In one implementation, the sine waves of different frequencies are controlled within the following range: an amplitude of 0.1 to 0.3 V, preferably 0.25 V; and a frequency of 1 to 200 KHZ, preferably 20 to 100 KHZ, with a preferred frequency interval of 20 KHZ, a number of frequencies n ≥ 2, and an impedance value measured at adjacent frequencies ≤ 5000 Ω, where an error will be reported and E-H will be displayed beyond the measured impedance value range. The measurement conditions for current measurement include a voltage of 0.15 to 0.45 V and a time of 5 to 60 s.

The total measurement time T for impedance values is 0.1 to 4 s. The measurement sequence follows an ascending order of frequencies, and the time for impedance collection at each frequency is t=T/n.
B. Concentration values of a metabolic index of a blood sample at different frequencies are obtained;
C. Weighted averaging is performed on the concentration values of the metabolic index of the same blood sample at the different frequencies to obtain a corrected concentration value of the metabolic index corresponding to the blood sample;

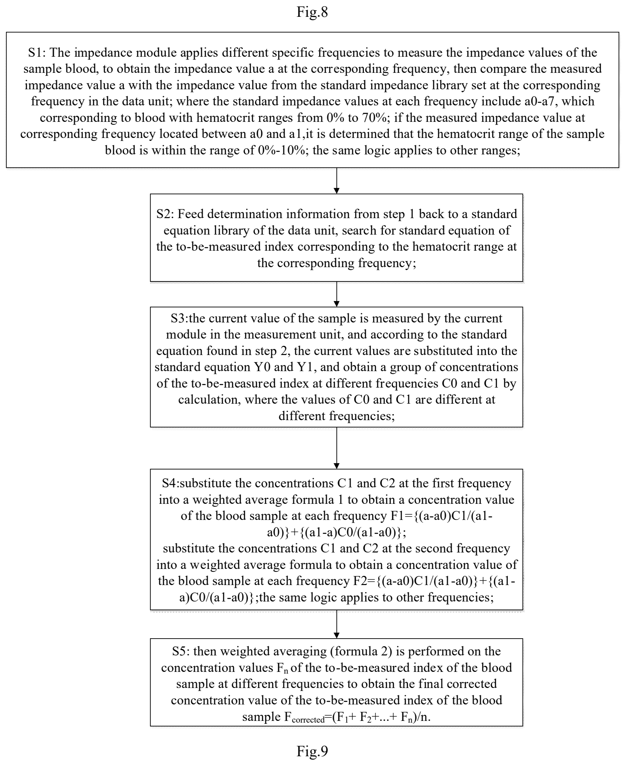

As shown in FIG. 9, the final corrected concentration value is F_{corrected}=(F₁+ F₂+...+ Fₙ)/n. Here, F₁ represents a concentration value of the metabolic index at the 1^{st} frequency, F₂ represents a concentration value of the metabolic index at the 2^{nd} frequency, and so on. Fₙ represents a concentration value of the metabolic index at the n^{th} frequency. In FIG. 9, a represents measured impedance values of the to-be-measured sample at different frequencies; a0 represents a standard impedance value with HCT of 0%; a1 represents a standard impedance value with HCT of 10%; C0 represents a concentration value calculated by substituting the current value of the to-be-measured blood into a standard equation with HCT of 0%; and C1 represents a concentration value calculated by substituting the current value of the to-be-measured blood into a blood glucose equation with HCT of 10%. The blood glucose equations Y0 and Y1 are quadratic equations with one variable, and their expression is Yi=a*xi²+b*x+c, where xi represents a current value of the to-be-measured index, Yi represents a concentration value of the to-be-measured index, and a, b, and c represent constant terms.

The concentration value of a certain metabolic index of the blood sample at a certain frequency is obtained through the method in Example 1.

In the embodiment of the present invention, the to-be-measured index may be a common electrochemical measurement index, such as blood glucose, uric acid, cholesterol, triglycerides, hemoglobin, β-hydroxybutyric acid, or creatinine.

In one implementation, after the impedance values of the measured sample at different frequencies are obtained, the impedance values are compared with the standard impedance library to determine whether the hematocrit range of the sample at different frequencies meets specified requirements, and if so, the operation continues; otherwise, an error is reported and E-H is displayed.

In this embodiment, the standard impedance library is established as follows: 5 male blood samples and 5 female blood samples are randomly drawn and prepared into 0%, 10%, 20%, 30%, 40%, 50%, 60%, and 70% blood samples respectively, the impedance values of each sample at different frequencies are measured with an instrument, and then the impedance values at corresponding hematocrit levels at each frequency are averaged to obtain the standard impedance library at different frequencies. Taking a frequency interval of 20 KHz and a frequency range of 10 to 90 KHZ as an example, the following standard impedance library is obtained, as shown in Table 5.

**Table 5 Standard impedance library in Example 3**

| Frequency | | 10 KHz | 30 KHz | 50 KHz | 70 KHz | 90 KHz |
|---|---|---|---|---|---|---|
| Serial numb er | Hematocrit of the blood sample | Impedance value (Ω) | | | | |
| 1 | 0% | 8000 | 7900 | 7750 | 7620 | 7300 |
| 2 | 10% | 9500 | 9350 | 9150 | 9050 | 8900 |
| 3 | 20% | 11500 | 11000 | 10600 | 10000 | 9800 |
| 4 | 30% | 14500 | 14000 | 13850 | 13550 | 13000 |
| 5 | 40% | 18000 | 17300 | 16950 | 16500 | 16000 |
| 6 | 50% | 22500 | 22000 | 21600 | 21000 | 20600 |
| 7 | 60% | 29500 | 28900 | 28100 | 27500 | 27000 |
| 8 | 70% | 40000 | 39400 | 38500 | 38000 | 37300 |

In this embodiment, the standard equation library is established as follows: a blood sample is randomly drawn and prepared into 8 blood samples with different hematocrit levels (0%, 10%, 20%, 30%, 40%, 50%, 60%, 70%); the blood sample at each hematocrit level is further divided into 8 equal parts and prepared into blood samples with different to-be-measured index concentrations; and the blood samples with different hematocrit levels and different to-be-measured index concentrations are calibrated (establishing a to-be-measured index concentration - current value relation curve) to obtain a group of standard equations (Y0, Y1, Y2, Y3, Y4, Y5, Y6, Y7).

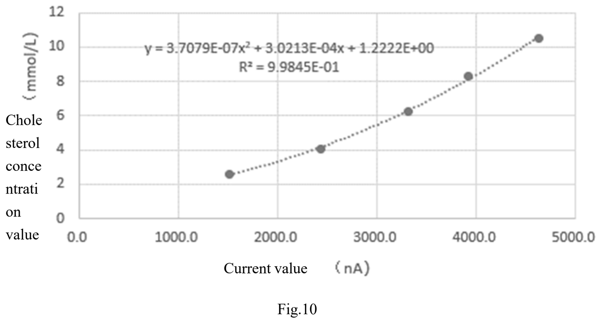

Taking the cholesterol measurement index as an example, a calibration process for a 40% hematocrit blood sample is as follows:
A blood sample is drawn and prepared into a 40% blood sample, the blood sample is divided into 2 equal parts, which are then prepared into 8 blood samples with different cholesterol concentrations respectively for calibration, and data is collected. The calibration by a current measurement module is shown in Table 6, and a standard equation is shown in FIG. 10. By analogy, a group of cholesterol standard equations for blood samples with different hematocrit levels can be obtained. The standard equation library is shown in Table 7.

**Table 6 Calibration by a cholesterol current measurement module (hematocrit 40%)**

| Blood sample | 1 | 2 | 3 | 4 | 5 |
|---|---|---|---|---|---|
| Biochemical value (mmol/L) | 2.55 | 4.02 | 6.25 | 8.26 | 10.46 |
| | 2.61 | 4.12 | 6.21 | 8.32 | 10.48 |
| Biochemical mean | 2.58 | 4.07 | 6.23 | 8.29 | 10.47 |

| Serial number | Current value (nA) | | | | |
|---|---|---|---|---|---|
| 1 | 1558 | 2482 | 3281 | 3910 | 4609 |
| 2 | 1508 | 2478 | 3387 | 3984 | 4555 |
| 3 | 1453 | 2377 | 3353 | 3943 | 4578 |
| 4 | 1567 | 2488 | 3286 | 3851 | 4669 |
| 5 | 1509 | 2449 | 3256 | 3902 | 4603 |
| 6 | 1474 | 2466 | 3317 | 3868 | 4664 |
| 7 | 1544 | 2461 | 3278 | 3991 | 4571 |
| 8 | 1563 | 2403 | 3325 | 3873 | 4672 |
| 9 | 1592 | 2430 | 3378 | 3941 | 4625 |
| 10 | 1540 | 2429 | 3387 | 3919 | 4679 |
| 11 | 1482 | 2363 | 3327 | 3978 | 4575 |
| 12 | 1506 | 2374 | 3378 | 3864 | 4672 |
| 13 | 1482 | 2387 | 3258 | 3991 | 4586 |
| 14 | 1463 | 2399 | 3306 | 3878 | 4643 |
| 15 | 1583 | 2497 | 3252 | 3866 | 4673 |
| 16 | 1497 | 2443 | 3329 | 3942 | 4652 |
| 17 | 1460 | 2485 | 3367 | 3989 | 4674 |
| 18 | 1462 | 2412 | 3297 | 3875 | 4626 |
| 19 | 1533 | 2475 | 3286 | 3867 | 4594 |
| 20 | 1587 | 2380 | 3289 | 3873 | 4617 |
| AV | 1518.2 | 2434.0 | 3316.8 | 3915.3 | 4626.7 |
| SD | 46.49 | 44.23 | 45.14 | 50.48 | 41.26 |
| CV | 3.06% | 1.82% | 1.36% | 1.29% | 0.89% |

**Table 7 Cholesterol standard equation library**

| Serial number | Hematocrit of the blood sample | Cholesterol standard equation |
|---|---|---|
| 1 | 0% | Y0 |
| 2 | 10% | Y1 |
| 3 | 20% | Y2 |
| 4 | 30% | Y3 |
| 5 | 40% | Y4 |
| 6 | 50% | Y5 |
| 7 | 60% | Y6 |
| 8 | 70% | Y7 |

In this embodiment, the specified requirements are as follows:
When n=2, the impedance values measured at two different frequencies are compared with the standard impedance library, indicating that the hematocrit ranges selected at the different frequencies need to be located within an adjacent or same hematocrit range; otherwise, an error will be reported and E-H will be displayed.
When n=3, the impedance values measured at 3 different frequencies are compared with the standard impedance library, indicating that at least two of the hematocrit ranges selected at the different frequencies are located within the same hematocrit range, and the other one is located within an adjacent hematocrit range; otherwise, an error will be reported and E-H will be displayed.
When n≥4, the impedance values measured at n different frequencies are compared with the standard impedance library, indicating that at least n-2 of the hematocrit ranges selected at the different frequencies are located within the same hematocrit range, and the others are located within an adjacent hematocrit range; otherwise, an error will be reported and E-H will be displayed.

### Example 4

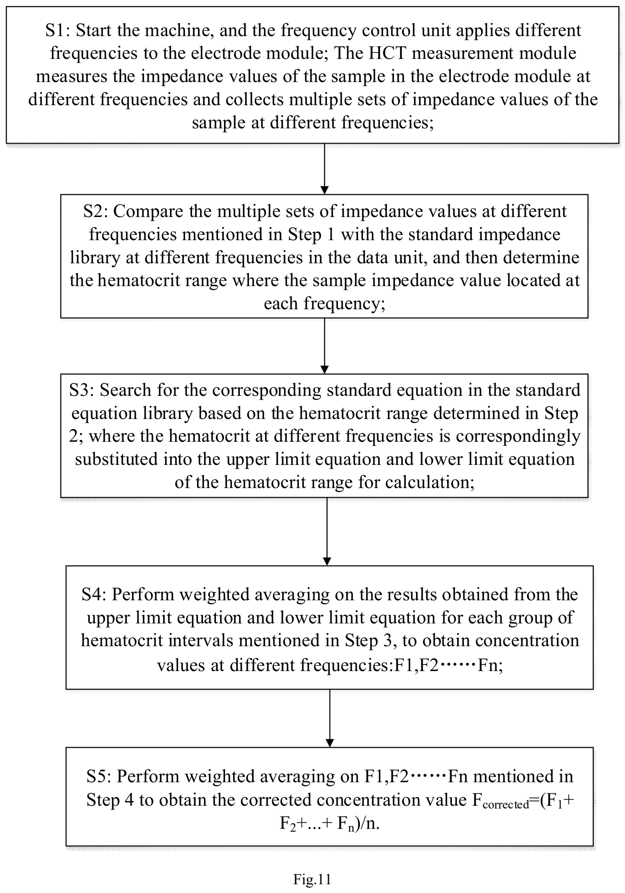

As shown in FIG. 11, this embodiment provides a metabolic index concentration measurement system corresponding to Example 3 above, including:
a frequency control unit, configured to apply a plurality of different frequencies to an electrode module of an electrochemical test strip;
a metabolic index concentration value obtaining unit (HCT measurement module), configured to obtain concentration values of a metabolic index of a blood sample at different frequencies; and
a corrected concentration value calculation unit, configured to perform weighted averaging on the concentration values of the metabolic index of the same blood sample at the different frequencies to obtain a corrected concentration value of the metabolic index corresponding to the blood sample.

The metabolic index concentration value obtaining unit in this embodiment is the metabolic index concentration measurement system in Example 2.

The measurement system in this embodiment further includes: a determination unit, configured to compare the impedance values at the different frequencies with a standard impedance library of different frequencies to determine whether the hematocrit range of the blood sample at the different frequencies meets set requirements, and if so, to send working instructions to the metabolic index concentration value obtaining unit, otherwise, issue an alarm instruction.

The calculation process/working process of each unit in this embodiment is identical to Example 3, and will not be elaborated here.

### Example 5

This embodiment provides an electrochemical measurement system, including:
an electrochemical test strip (test strip); and
a linker, configured to transmit corresponding reaction signals generated by a sample on the electrochemical test strip to a measurement unit, so as to achieve accurate measurement of blood glucose concentration and uric acid concentration.

The measurement unit in this embodiment is the metabolic index concentration measurement system in Example 2 and/or Example 4 above.

When the electrochemical measurement system in this embodiment is used for measurement, blood is first drawn into the electrochemical test strip, the linker transmits reaction signals to the metabolic index concentration measurement system, each measurement module in the metabolic index concentration measurement system carries out relevant measurement and transmits the measured value to the calculation unit, the calculation unit calculates a blood glucose concentration and a uric acid concentration, and finally, the display unit displays a blood glucose concentration value and a uric acid concentration value.

### Example 6

Blood glucose and uric acid test strips (electrochemical test strips) were prepared, an electrochemical measurement system was constructed, and the repeatability, accuracy, and hematocrit correction accuracy of the electrochemical measurement system were measured by using the method in Example 1 above.

Firstly, measurement condition parameters were set: the measurement conditions for the HCT measurement module included an amplitude of 0.1 V, a frequency of 1 KHz, and a measurement time of 0.1 s; the measurement conditions for the blood glucose measurement module included a voltage of 0.2 V and a time of 5 s; and the measurement conditions for the uric acid measurement module included a voltage of 0.45 V and a pressure time of 10 s.

1. The repeatability of the electrochemical measurement system was tested: fasting fresh venous whole blood was collected from blood sample providers with a hematocrit level of 0.35 L/L to 0.50 L/L (35% to 50%) and transferred into test tubes containing a heparin sodium anticoagulant to avoid hemolysis. The samples were placed at room temperature (23°C±5°C) for equilibration for at least 30 minutes until the sample temperature reached room temperature ±2°C. Repeatability test was carried out by using 5 venous whole blood samples within a blood glucose concentration range in Table 8 and a uric acid concentration range in Table 9. Before testing, each sample should be gently turned over to ensure thorough mixing. Then, the test was repeated 20 times respectively on two supporting instruments (which were used to implement the function of the metabolic index concentration measurement system in Example 2 and were used with blood glucose and uric acid test strips) to calculate standard deviations (SD) and coefficients of variation (CV) of results. The repeatability results of blood glucose concentration are shown in Table 10, and the repeatability results of uric acid concentration are shown in Table 11.

**Table 8 Blood glucose concentration range for repeatability test**

| Sample number | Blood glucose concentration [mmol/L (mg/dL)] |
|---|---|
| 1 | 1.67-2.78 (30-50) |
| 2 | 2.83-6.11 (51-110) |
| 3 | 6.16-8.33 (111-150) |
| 4 | 8.38-13.88 (151-250) |
| 5 | 13.93-22.20 (251-400) |

**Table 9 Uric acid concentration range for repeatability test**

| Sample number | Uric acid concentration [umol/L (mg/dL)] |
|---|---|
| 1 | 180-290 (3-4.8) |
| 2 | 300-510 (5-8.5) |
| 3 | 520-720 (8.7-12) |
| 4 | 730-900 (12.2-15) |
| 5 | 910-1190 (15.2-20) |

**Table 10 Repeatability of blood glucose concentration**

| Blood sample | 1 | 2 | 3 | 4 | 5 |
|---|---|---|---|---|---|
| Biochemi cal value (mmol/L) | 2.35 | 4.25 | 7.54 | 11.45 | 17.2 |
| | 2.4 | 4.29 | 7.42 | 11.36 | 17.1 |
| Biochemi cal mean | 2.38 | 4.27 | 7.48 | 11.41 | 17.15 |

| Serial number | Blood glucose concentration value (mmol/L) | | | | |
|---|---|---|---|---|---|
| 1 | 2.3 | 4 | 7.2 | 11.6 | 16.5 |
| 2 | 2.2 | 3.9 | 7.5 | 11.4 | 16.5 |
| 3 | 2.1 | 4 | 7.6 | 11 | 16.3 |
| 4 | 2.3 | 3.9 | 7.5 | 11.3 | 17.7 |
| 5 | 2.3 | 4.1 | 7.6 | 11.4 | 16.2 |
| 6 | 2.3 | 4.1 | 7.1 | 10.9 | 16.6 |
| 7 | 2.2 | 4.2 | 7.2 | 11.3 | 16.8 |
| 8 | 2.2 | 4.1 | 7.4 | 11.6 | 17 |
| 9 | 2.3 | 4.2 | 7.3 | 11.9 | 17.2 |
| 10 | 2.3 | 4.2 | 7.2 | 11.6 | 17.5 |
| 11 | 2.2 | 3.9 | 7.4 | 11 | 17 |
| 12 | 2.3 | 4.3 | 7.5 | 11.1 | 17.2 |
| 13 | 2.2 | 4.1 | 7.2 | 11.3 | 16.7 |
| 14 | 2.2 | 4 | 7.2 | 11.4 | 16.6 |
| 15 | 2.3 | 4.2 | 7.2 | 11.3 | 17.3 |
| 16 | 2.2 | 4.1 | 7.6 | 10.7 | 16.1 |
| 17 | 2.3 | 4.1 | 7.4 | 11.3 | 16.2 |
| 18 | 2.2 | 3.9 | 7.8 | 12 | 17 |
| 19 | 2.5 | 4.2 | 7.8 | 12.2 | 16.7 |
| 20 | 2.2 | 3.8 | 7.4 | 11.5 | 17.2 |
| AV | 2.3 | 4.1 | 7.4 | 11.4 | 16.8 |
| SD | 0.083 | 0.135 | 0.206 | 0.368 | 0.452 |
| CV | 3.66% | 3.32% | 2.79% | 3.23% | 2.69% |

**Table 11 Repeatability of uric acid concentration**

| Blood sample | 1 | 2 | 3 | 4 | 5 |
|---|---|---|---|---|---|
| Biochemi cal value (umol/L) | 224 | 423 | 606 | 779 | 965 |
| | 223 | 427 | 595 | 778 | 967 |
| Biochemi cal mean | 224 | 425 | 601 | 779 | 966 |

| Serial number | Uric acid concentration value (umol/L) | | | | |
|---|---|---|---|---|---|
| 1 | 210 | 392 | 550 | 754 | 955 |
| 2 | 202 | 420 | 545 | 745 | 970 |
| 3 | 208 | 421 | 540 | 739 | 980 |
| 4 | 210 | 410 | 551 | 746 | 961 |
| 5 | 205 | 421 | 537 | 770 | 920 |
| 6 | 212 | 420 | 552 | 757 | 995 |
| 7 | 213 | 430 | 570 | 760 | 990 |
| 8 | 211 | 412 | 520 | 720 | 970 |
| 9 | 203 | 400 | 510 | 772 | 980 |
| 10 | 210 | 390 | 543 | 760 | 960 |
| 11 | 206 | 420 | 545 | 770 | 960 |
| 12 | 200 | 410 | 556 | 750 | 952 |
| 13 | 210 | 410 | 540 | 790 | 956 |
| 14 | 222 | 420 | 535 | 760 | 966 |
| 15 | 200 | 398 | 551 | 770 | 930 |
| 16 | 203 | 400 | 530 | 750 | 960 |
| 17 | 201 | 400 | 553 | 760 | 970 |
| 18 | 215 | 410 | 533 | 760 | 950 |
| 19 | 208 | 430 | 551 | 765 | 980 |
| 20 | 210 | 430 | 553 | 769 | 978 |
| AV | 207.95 | 412.20 | 543.25 | 758.35 | 964.15 |
| SD | 5.58 | 12.39 | 13.44 | 14.69 | 18.28 |
| CV | 2.68% | 3.01% | 2.47% | 1.94% | 1.90% |

The above repeatability verification results show that the testing method in the embodiments of the present invention can control the repeatability of blood glucose and uric acid test strips within 4.0%, demonstrating excellent measurement repeatability.

### 2. Verification on HCT accuracy of the electrochemical measurement system:

Fresh fasting venous whole blood was collected from blood sample providers with a hematocrit level of 0.35 L/L to 0.50 L/L (35% to 50%) within 24 hours and transferred into test tubes containing a heparin sodium anticoagulant to avoid hemolysis. According to the blood glucose concentration range in Table 12, three blood samples with different blood glucose concentrations were prepared, then the blood sample of each concentration was divided into 5 equal parts and centrifuged, the hematocrit of each part of blood was adjusted to 10%, 25%, 42%, 55%, and 70% respectively, and the samples were placed at room temperature (23°C±5°C) for equilibration for at least 30 minutes; according to the uric acid concentration range in Table 13, three blood samples with different uric acid concentrations were prepared, then the blood sample of each concentration was divided into 5 equal parts and centrifuged, the hematocrit of each part of blood was adjusted to 10%, 25%, 42%, 55%, and 70% respectively, and the samples were placed at room temperature (23°C±5°C) for equilibration for at least 30 minutes; after the blood sample of each concentration and each hematocrit was shaken thoroughly, the blood samples were repeatedly measured 10 times by using 2 supporting instruments, and an average value of the measurement results was calculated; finally, each blood sample was measured twice by using a biochemical analyzer, an average value of the measurement results was calculated, and a deviation between the average values of the measurement results of the blood samples from the supporting instruments and the biochemical analyzer was calculated; and a deviation between the average value of the measurement results of the hematocrit blood samples and the average value of the measurement results of the 40% hematocrit blood samples was calculated. The measurement results of blood glucose HCT accuracy are shown in Table 14, and the measurement results of uric acid HCT accuracy are shown in Table 15.

**Table 12: Blood glucose concentration range for hematocrit effect**

| Sample number | Blood glucose concentration /[mmol/L (mg/dL)] |
|---|---|
| 1 | [1.67, 2.78]mmol/L{(30-50]mg/dL} |
| 2 | [5.33, 7.99]mmol/L{(96-144]mg/dL} |
| 3 | [15.54, 23.31]mmol/L {(280-420]mg/dL} |

**Table 13 Uric acid concentration range for hematocrit effect**

| Sample number | Uric acid concentration [umol/L (mg/dL)] |
|---|---|
| 1 | 180-290 (3-4.8) |
| 2 | 350-700 (5-10) |
| 3 | 750-900 (12.5-15) |

**Table 14 Verification report on blood glucose concentration HCT accuracy**

| Blood sample | 1 | | | | | 2 | | | | | 3 | | | | |
|---|---|---|---|---|---|---|---|---|---|---|---|---|---|---|---|
| Biochemical value (mmol/L) | 1.9 7 | 1.87 | 1.7 8 | 1.5 1 | 1.5 1 | 6.4 0 | 6.3 6 | 6.6 8 | 6.2 8 | 6.3 7 | 20. 18 | 20.0 0 | 20. 09 | 20. 20 | 20.0 7 |
| Biochemical mean | 1.9 8 | 1.88 | 1.6 7 | 1.4 2 | 1.3 6 | 6.4 7 | 6.3 3 | 6.5 6 | 6.2 8 | 6.3 7 | 20. 20 | 20.1 0 | 20. 20 | 19. 93 | 19.8 0 |
| Blood HCT (%) | 10 % | 25% | 42 % | 55 % | 70 % | 10 % | 25 % | 42 % | 55 % | 70 % | 10 % | 25% | 42 % | 55 % | 70% |

| Serial number | Blood glucose concentration value (mmol/L) | | | | | | | | | | | | | | |
|---|---|---|---|---|---|---|---|---|---|---|---|---|---|---|---|
| 1 | 2.1 | 2.1 | 2.2 | 2.0 | 1.8 | 6.3 | 6.2 | 6.7 | 6.5 | 6.4 | 19. 0 | 18.9 | 20. 6 | 19.4 | 19.2 |
| 2 | 2.1 | 2.1 | 2.2 | 2.0 | 2.0 | 6.4 | 6.6 | 6.6 | 6.6 | 6.4 | 19. 9 | 18.8 | 19. 8 | 19.6 | 19.4 |
| 3 | 2.2 | 2.2 | 2.4 | 1.9 | 1.9 | 6.3 | 6.6 | 6.3 | 6.5 | 6.4 | 19. 2 | 19.4 | 20. 9 | 19.7 | 19.7 |
| 4 | 2.2 | 2.0 | 2.0 | 2.0 | 1.7 | 6.3 | 6.8 | 6.1 | 7.4 | 6.1 | 19. 6 | 19.3 | 21. 0 | 20.3 | 19.6 |
| 5 | 2.2 | 2.1 | 2.6 | 1.8 | 1.6 | 6.4 | 6.8 | 6.5 | 6.6 | 6.5 | 19. 8 | 19.7 | 20. 5 | 20.0 | 19.5 |
| 6 | 2.0 | 2.0 | 2.2 | 2.1 | 1.9 | 6.6 | 6.6 | 6.9 | 6.8 | 6.4 | 20. 2 | 20.4 | 21. 5 | 19.9 | 19.9 |
| 7 | 2.2 | 2.0 | 2.4 | 1.8 | 2.1 | 6.5 | 6.9 | 7.0 | 7.4 | 6.3 | 20. 4 | 19.8 | 21. 3 | 19.9 | 19.4 |
| 8 | 2.2 | 2.2 | 2.6 | 2.3 | 1.9 | 6.2 | 7.0 | 7.1 | 7.1 | 6.2 | 22. 8 | 20.3 | 21. 8 | 19.5 | 19.6 |
| 9 | 2.1 | 2.0 | 2.4 | 2.2 | 2.0 | 6.6 | 7.0 | 7.5 | 7.8 | 5.9 | 20. 1 | 20.1 | 21. 6 | 19.7 | 19.0 |
| 10 | 2.1 | 2.1 | 2.1 | 2.1 | 2.0 | 6.4 | 6.9 | 7.1 | 7.9 | 6.2 | 20. 2 | 19.8 | 21. 6 | 20.0 | 21.8 |
| Measured average value | 2.1 | 2.1 | 2.3 | 2.0 | 1.9 | 6.4 | 6.7 | 6.8 | 7.1 | 6.3 | 20. 1 | 19.7 | 21. 1 | 19.8 | 19.7 |
| Absolute deviation from 40% HCT blood sample | - 0.1 7 | 0.23 | / | - 0.2 9 | - 0.4 2 | / | | | | | | | | | |
| Relative deviation from 40% HCT blood sample | / | | | | | - 5.6 0% | - 0.5 9% | / | 4.1 3% | - 7.3 7% | - 4.4 6% | - 6.70 % | / | - 5.98 % | - 6.41 % |
| Absolute deviation from the biochemical analyzer | 0.1 7 | 0.21 | 0.5 9 | 0.5 6 | 0.4 6 | / | | | | | | | | | |
| Relative deviation from the biochemical analyzer | / | | | | | - 0.5 4% | 6.2 3% | 2.4 2% | 12. 42 % | - 1.4 1% | - 0.35 % | - 2.00 % | 4.5 4% | - 1.32 % | - 1.1 3% |

**Table 15 Verification report on uric acid concentration HCT accuracy**

| Blood sample | 1 | | | | | 2 | | | | | 3 | | | | | | | |
|---|---|---|---|---|---|---|---|---|---|---|---|---|---|---|---|---|---|---|
| Biochemic al value (umol/L) | 232 | 230 | 235 | 236 | 241 | 51 9 | 52 2 | 52 3 | 52 8 | 52 7 | 84 9 | 84 5 | 85 0 | | 855 | | 85 7 | |
| Biochemic al mean | 232 | 233 | 239 | 241 | 245 | 52 2 | 52 0 | 53 2 | 52 3 | 52 5 | 84 4 | 84 7 | 85 2 | | 853 | | 85 9 | |
| Blood HCT (%) | 10% | 25% | 42 % | 55% | 70 % | 10 % | 25 % | 42 % | 55 % | 70 % | 10 % | 25 % | 42 % | | 55% | | 70 % | |

| Serial number | Uric acid concentration value (umol/L) | | | | | | | | | | | | | | | | | |
|---|---|---|---|---|---|---|---|---|---|---|---|---|---|---|---|---|---|---|
| 1 | 250 | 249 | 245 | 239 | 230 | 52 9 | 54 0 | 53 5 | 53 5 | 53 5 | 83 9 | 84 7 | 84 5 | | 847 | | 84 4 | |
| 2 | 243 | 252 | 246 | 238 | 234 | 53 2 | 53 2 | 54 5 | 54 5 | 54 5 | 83 3 | 85 0 | 84 9 | | 844 | | 85 5 | |
| 3 | 256 | 250 | 247 | 242 | 239 | 53 1 | 53 3 | 54 4 | 54 4 | 54 4 | 83 4 | 85 2 | 85 3 | | 850 | | 85 4 | |
| 4 | 242 | 256 | 252 | 241 | 241 | 52 7 | 53 7 | 55 1 | 55 1 | 55 1 | 85 6 | 85 1 | 85 5 | | 851 | | 85 3 | |
| 5 | 246 | 241 | 234 | 243 | 242 | 53 6 | 53 7 | 55 2 | 55 2 | 55 2 | 84 6 | 86 1 | 85 7 | | 861 | | 85 6 | |
| 6 | 239 | 243 | 239 | 235 | 238 | 53 4 | 53 4 | 53 6 | 53 6 | 53 6 | 84 8 | 84 9 | 84 5 | | 851 | | 85 0 | |
| 7 | 241 | 247 | 241 | 233 | 237 | 52 9 | 53 3 | 55 7 | 55 7 | 55 7 | 84 6 | 85 4 | 85 3 | | 850 | | 85 0 | |
| 8 | 249 | 244 | 245 | 237 | 241 | 54 1 | 53 2 | 55 6 | 55 6 | 55 6 | 84 3 | 85 1 | 85 4 | | 847 | | 85 1 | |
| 9 | 244 | 243 | 244 | 240 | 240 | 53 7 | 54 1 | 54 7 | 54 7 | 54 7 | 83 9 | 84 9 | 84 7 | | 849 | | 84 9 | |
| 10 | 244 | 240 | 242 | 242 | 243 | 53 0 | 54 0 | 54 5 | 54 5 | 54 5 | 84 0 | 84 7 | 84 6 | | 849 | | 85 7 | |
| Measured average value | 245. 4 | 246. 5 | | 243. 5 | | 239. 0 | | 238 .5 | 53 2.6 | 53 5.9 | 54 6.8 | 54 6.8 | 54 6.8 | 84 2.4 | 85 1.1 | 85 0.4 | 849. 9 | 85 1.9 |
| Absolute deviation from 40% HCT blood sample | 1.90 | 3.00 | | / | | 4.50 | | - 5.0 0 | / | | | | | | | | | |
| Relative deviation from 40% HCT blood sample (%) | / | | | | | | | | - 2.6 0% | - 1.9 9% | / | 0.0 0% | 0.0 0% | - 0.9 4% | 0.0 8% | / | - 0.06 % | 0.1 8% |
| Absolute deviation from the biochemic al analyzer | 13.40 | | 15.0 0 | | 6.5 0 | | 0.5 0 | 4. 5 0 | / | | | | | | | | | |
| Relative deviation from the biochemic al analyzer (%) | / | | | | | | | | 2.3 2 | 2.8 6 | 3.6 6 | 4.0 5 | 3.9 5 | 0.4 8 | 0.6 0 | 0.0 7 | 0.4 8 | 0.7 1 |

The above accuracy verification results show that the measurement method in the embodiment of the present invention can ensure that, on the one hand, the test results of the blood samples within 10-70% hematocrit levels have relatively small deviations from those within intermediate 40% hematocrit level (the relative deviation of blood glucose was controlled within 7.5%, and the relative deviation of uric acid was controlled within 3.0%), and on the other hand, the test results of different HCT blood samples have relatively small deviations from biochemical values (the relative deviation of blood glucose was controlled within 13%, and the relative deviation of uric acid was controlled within 5.0%). Therefore, the measurement method in the embodiment of the present invention can implement HCT correction of blood glucose and uric acid test results, making the product applicable to a broader population.

### Example 7

Cholesterol test strips were prepared by using the measurement method in Example 3 of the present invention to test the repeatability, accuracy, and hematocrit correction accuracy of the test strips.

First, parameters were set: the measurement conditions for the HCT measurement module included an amplitude of 0.3 V, three different frequencies (10 KHz, 30 KHz, and 90 KHz), and a measurement time of 1.2 s; and the measurement conditions for the current measurement module included a voltage of 0.3 V and a time of 50 s.
1. The repeatability of the test strips was tested: fasting fresh venous whole blood was collected from blood sample providers with a hematocrit level of 0.35 L/L to 0.50 L/L (35% to 50%) and transferred into test tubes containing a heparin sodium anticoagulant to avoid hemolysis. The samples were placed at room temperature (23°C±5°C) for equilibration for at least 30 minutes until the sample temperature reached room temperature ±2°C. Repeatability test was carried out by using 5 venous whole blood samples within a cholesterol concentration range in Table 16. Before testing, each sample should be gently turned over to ensure thorough mixing. Then, the test was repeated 20 times respectively on two supporting instruments (which were the electrochemical measurement system in Example 4, and the measurement unit in the electrochemical measurement system was the metabolic index concentration measurement system in Example 2) to calculate a standard deviation (SD) and a coefficient of variation (CV) of results. The repeatability results of cholesterol concentration are shown in Table 17.

**Table 16 Cholesterol concentration range for repeatability test**

| Sample number | Cholesterol concentration [mmol/L] |
|---|---|
| 1 | 2.3-3.5 |
| 2 | 3.6-4.9 |
| 3 | 5.0-6.5 |
| 4 | 6.6-8.9 |
| 5 | 9.0-12.0 |

**Table 17: Verification report on repeatability and accuracy of cholesterol test strips**

| Blood sample | 1 | 2 | 3 | 4 | 5 |
|---|---|---|---|---|---|
| Biochemical | 2.6 | 4.05 | 6.4 | 8.12 | 11.21 |
| | 2.61 | 4.1 | 6.35 | 8.25 | 11.16 |
| Biochemical AV | 2.605 | 4.075 | 6.375 | 8.185 | 11.185 |

| Serial number | | | | | |
|---|---|---|---|---|---|
| 1 | 2.4 | 4.1 | 6.4 | 8.2 | 11.3 |
| 2 | 2.3 | 4.2 | 6.5 | 8.3 | 11.4 |
| 3 | 2.3 | 3.9 | 6.4 | 8.4 | 11.5 |
| 4 | 2.5 | 3.9 | 6.5 | 8.4 | 11.6 |
| 5 | 2.3 | 4.1 | 6.6 | 8.6 | 11.8 |
| 6 | 2.4 | 4.1 | 6.6 | 8.3 | 12.2 |
| 7 | 2.5 | 4.2 | 6.7 | 8.4 | 12 |
| 8 | 2.6 | 4.1 | 6.4 | 8.3 | 11.7 |
| 9 | 2.3 | 4.2 | 6.5 | 8.4 | 11.8 |
| 10 | 2.5 | 4.2 | 6.4 | 8.6 | 11.9 |
| 11 | 2.2 | 3.9 | 6.6 | 8.4 | 11.4 |
| 12 | 2.6 | 4.3 | 6.5 | 8.3 | 11.3 |
| 13 | 2.3 | 4.1 | 6.4 | 8.6 | 11.4 |
| 14 | 2.4 | 4 | 6.6 | 8.7 | 11.5 |
| 15 | 2.6 | 4.2 | 6.7 | 8.4 | 11.6 |
| 16 | 2.5 | 4.1 | 6.7 | 8.5 | 11.4 |
| 17 | 2.6 | 4.1 | 6.6 | 8.6 | 11.6 |
| 18 | 2.4 | 3.9 | 6.3 | 8.7 | 12.3 |
| 19 | 2.6 | 4.2 | 6.4 | 8.8 | 12.1 |
| 20 | 2.3 | 3.8 | 6.5 | 8.6 | 12 |
| AV | 2.4 | 4.1 | 6.5 | 8.5 | 11.7 |
| SD | 0.130 | 0.136 | 0.118 | 0.165 | 0.308 |
| CV | / | 3.34% | 1.81% | 1.95% | 2.63% |
| Absolute deviation | -0.2 | 0.0 | 0.1 | 0.3 | 0.5 |
| Relative deviation | / | 0.1% | 2.2% | 3.5% | 4.5% |

The verification results of repeatability and accuracy show that the measurement method in Example 3 of the present invention can control the repeatability of cholesterol test strips within 4.0% and control the relative deviation within 5.0%, demonstrating excellent measurement repeatability and accuracy.

### 2. Verification on HCT accuracy of test strips:

Fresh fasting venous whole blood was collected from blood sample providers with a hematocrit level of 0.35 L/L to 0.50 L/L (35% to 50%) within 24 hours and transferred into test tubes containing a heparin sodium anticoagulant to avoid hemolysis. According to the cholesterol concentration range in Table 18, three blood samples with different cholesterol concentrations were prepared, then the blood sample of each concentration was divided into 5 equal parts and centrifuged, the hematocrit of each part of blood was adjusted to 0%, 20%, 42%, 55%, and 70% respectively, and the samples were placed at room temperature (23°C+5°C) for equilibration for at least 30 minutes; after the blood sample of each concentration and each hematocrit was shaken thoroughly, the blood samples were repeatedly measured 10 times by using 2 supporting instruments ((which were the electrochemical measurement system in Example 4, and the measurement unit in the electrochemical measurement system was the metabolic index concentration measurement system in Example 2), and an average value of the measurement results was calculated; finally, each blood sample was measured twice by using a biochemical analyzer, an average value of the measurement results was calculated, and a deviation between the average values of the measurement results of the blood samples from the supporting instruments and the biochemical analyzer was calculated; and a deviation between the average value of the measurement results of the hematocrit blood samples and the average value of the measurement results of the 40% hematocrit blood samples was calculated. The measurement results of cholesterol HCT accuracy are shown in Table 19.

**Table 18 Cholesterol concentration range for hematocrit effect**

| Sample number | Cholesterol concentration /[mmol/L] |
|---|---|
| 1 | [2.3, 3.5] mmol/L |
| 2 | [4.0, 6.0] mmol/L |
| 3 | [6.5, 8.5] mmol/L |

**Table 19: Verification report on cholesterol concentration HCT accuracy**

| Blood sample | 1 | | | | | 2 | | | | | 3 | | | | |
|---|---|---|---|---|---|---|---|---|---|---|---|---|---|---|---|
| Biochemical value (mmol/L) | 2.8 3 | 2. 78 | 2. 84 | 2. 76 | 2. 69 | 5.2 3 | 5.1 6 | 5.2 0 | 5.1 8 | 5.2 2 | 7.2 0 | 7.2 2 | 7.2 4 | 7.2 3 | 7.2 2 |
| | 2.8 0 | 2. 79 | 2. 81 | 2. 74 | 2. 68 | 5.2 6 | 5.1 2 | 5.2 1 | 5.2 0 | 5.2 0 | 7.2 3 | 7.2 0 | 7.2 3 | 7.1 8 | 7.1 9 |
| Biochemical mean | 2.8 2 | 2. 79 | 2. 83 | 2. 75 | 2. 69 | 5.2 5 | 5.1 4 | 5.2 1 | 5.1 9 | 5.2 1 | 7.2 2 | 7.2 1 | 7.2 4 | 7.2 1 | 7.2 1 |
| Blood HCT (%) | 0 | 20 | 42 | 55 | 70 | 0 | 20 | 42 | 55 | 70 | 0 | 20 | 42 | 55 | 70 |

| Serial number | Concentration value (mmol/L) | | | | | | | | | | | | | | |
|---|---|---|---|---|---|---|---|---|---|---|---|---|---|---|---|
| 1 | 2.6 | 2. 6 | 2. 6 | 2. 6 | 2. 7 | 5.7 | 5.5 | 5.6 | 5.3 | 5.4 | 7.5 | 7.5 | 7.4 | 7.6 | 7.3 |
| 2 | 2.6 | 2. 6 | 2. 6 | 2. 5 | 2. 6 | 5.5 | 5.6 | 5.6 | 5.5 | 5.4 | 7.6 | 7.6 | 7.6 | 7.3 | 7.4 |
| 3 | 2.5 | 2. 7 | 2. 5 | 2. 7 | 2. 6 | 5.6 | 5.6 | 5.4 | 5.6 | 5.5 | 7.5 | 7.4 | 7.7 | 7.3 | 7.7 |
| 4 | 2.7 | 2. 5 | 2. 5 | 2. 7 | 2. 6 | 5.4 | 5.5 | 5.3 | 5.4 | 5.6 | 7.6 | 7.5 | 7.6 | 7.4 | 7.6 |
| 5 | 2.5 | 2. 6 | 2. 4 | 2. 5 | 2. 5 | 5.6 | 5.5 | 5.3 | 5.5 | 5.5 | 7.4 | 7.6 | 7.3 | 7.7 | 7.6 |
| 6 | 2.5 | 2. 7 | 2. 4 | 2. 4 | 2. 8 | 5.3 | 5.4 | 5.4 | 5.5 | 5.4 | 7.6 | 7.4 | 7.5 | 7.7 | 7.3 |
| 7 | 2.6 | 2. 7 | 2. 6 | 2. 5 | 2. 7 | 5.7 | 5.6 | 5.5 | 5.3 | 5.5 | 7.4 | 7.5 | 7.7 | 7.6 | 7.4 |
| 8 | 2.7 | 2. 7 | 2. 6 | 2. 6 | 2. 7 | 5.5 | 5.5 | 5.3 | 5.5 | 5.3 | 7.6 | 7.6 | 7.4 | 7.3 | 7.6 |
| 9 | 2.6 | 2. 6 | 2. 6 | 2. 6 | 2. 6 | 5.5 | 5.5 | 5.5 | 5.5 | 5.4 | 7.4 | 7.5 | 7.4 | 7.5 | 7.3 |
| 10 | 2.5 | 2. 6 | 2. 5 | 2. 4 | 2. 7 | 5.7 | 5.5 | 5.6 | 5.5 | 5.4 | 7.3 | 7.5 | 7.7 | 7.5 | 7.7 |
| Measured average value | 2.6 | 2. 6 | 2. 5 | 2. 6 | 2. 6 | 5.6 | 5.5 | 5.4 | 5.5 | 5.4 | 7.5 | 7.5 | 7.5 | 7.5 | 7.5 |
| Absolute deviation from 40% HCT blood sample | 0.0 6 | 0. 11 | / | 0. 04 | 0. 13 | | | | | | | | | | |
| Relative deviation from 40% HCT blood sample | | | | | | 2.3 % | 1.8 % | / | 0.3 % | 0.0 % | 0.6 % | 0.5 % | / | 0.7 % | 0.8 % |
| Absolute deviation from the biochemical analyzer | 0.2 3 | 0. 16 | 0. 30 | 0. 19 | 0. 04 | | | | | | | | | | |
| Relative deviation from the biochemical analyzer | | | | | | 6.1 % | 7.7 % | 4.5 % | 5.2 % | 4.4 % | 3.9 % | 4.1 % | 4.3 % | 4.0 % | 3.9 % |

The accuracy verification results show that the measurement method in Example 3 of the present invention can ensure that, on the one hand, the test results of the blood samples within 0-70% hematocrit levels have relatively small deviations from those within intermediate 40% hematocrit level (the relative deviation was controlled within 5.0%), and on the other hand, the test results of different HCT blood samples have relatively small deviations from biochemical values (the relative deviation was controlled within 6.5%). Therefore, the measurement method in Example 3 of the present invention can implement HCT correction of cholesterol test results, making the product applicable to a broader population.

By analogy, this HCT correction system can be applied to other common electrochemical measurement indexes, such as blood glucose, uric acid, triglycerides, hemoglobin, β-hydroxybutyric acid, or creatinine.

### Comparative Example 1

This example employed the electrochemical measurement system identical to Example 6, where the metabolic index concentration measurement system did not include the data unit or the weighted averaging step, only 40% blood samples were used for calibration, and only one linear blood glucose equation and one uric acid concentration equation were adopted. The accuracy of test strips was evaluated by using the accuracy verification method in Example 3. The blood glucose test results are shown in Table 20, and the uric acid test results are shown in Table 21.

**Table 20 Verification report on blood glucose concentration accuracy**

| Blood sample | 1 | | | | | 2 | | | | | 3 | | | | |
|---|---|---|---|---|---|---|---|---|---|---|---|---|---|---|---|
| Bioche mical value (mmol/ L) | 1. 97 | 1. 87 | 1. 78 | 1. 51 | 1. 51 | 6.40 | 6.36 | 6.6 8 | 6.28 | 6.37 | 20.1 8 | 20.0 0 | 20. 09 | 20.2 0 | 20.0 7 |
| Bioche mical mean | 1. 98 | 1. 88 | 1. 67 | 1. 42 | 1. 36 | 6.47 | 6.33 | 6.5 6 | 6.28 | 6.37 | 20.2 0 | 20.1 0 | 20. 20 | 19.9 3 | 19.8 0 |
| Blood HCT (%) | 10 % | 25 % | 42 % | 55 % | 70 % | 10% | 25% | 42 % | 55% | 70% | 10% | 25% | 42 % | 55% | 70% |

| Serial number | Blood glucose concentration value (mmol/L) | | | | | | | | | | | | | | |
|---|---|---|---|---|---|---|---|---|---|---|---|---|---|---|---|
| 1 | 3. 6 | 2. 8 | 2. 1 | 1. 5 | 1. 0 | 8.1 | 7.7 | 6.7 | 5.7 | 4.9 | 25.6 | 23.0 | 20. 6 | 17.5 | 14.6 |
| 2 | 3. 5 | 2. 9 | 2. 2 | 1. 4 | 1. 1 | 8.0 | 7.6 | 6.6 | 5.6 | 4.8 | 25.4 | 22.2 | 19. 8 | 17.6 | 14.9 |
| 3 | 3. 6 | 2. 7 | 2. 3 | 1. 3 | 1. 1 | 8.2 | 7.5 | 6.3 | 5.3 | 4.7 | 26.1 | 23.1 | 20. 9 | 17.2 | 14.7 |
| 4 | 3. 7 | 2. 9 | 2. 0 | 1. 3 | 1. 3 | 8.3 | 7.6 | 6.1 | 5.4 | 4.8 | 26.8 | 22.3 | 21. 0 | 16.9 | 15.1 |
| 5 | 3. 8 | 3. 0 | 2. 5 | 1. 4 | 1. 2 | 8.1 | 7.4 | 6.5 | 5.6 | 4.3 | 26.7 | 22.4 | 20. 5 | 16.5 | 14.9 |
| 6 | 3. 4 | 3. 1 | 2. 2 | 1. 3 | 1. 1 | 8.2 | 7.4 | 6.9 | 5.5 | 4.7 | 26.7 | 22.5 | 21. 5 | 16.3 | 14.7 |
| 7 | 3. 5 | 3. 2 | 2. 4 | 1. 5 | 1. 1 | 8.3 | 7.7 | 7.0 | 5.5 | 4.6 | 26.5 | 23.9 | 21. 3 | 15.9 | 15.2 |
| 8 | 3. 6 | 3. 0 | 2. 3 | 1. 6 | 1. 4 | 8.2 | 7.7 | 7.1 | 5.6 | 4.5 | 26.0 | 24.2 | 21. 8 | 16.6 | 14.9 |
| 9 | 3. 4 | 3. 1 | 2. 2 | 1. 4 | 1. 2 | 8.1 | 7.5 | 7.5 | 5.5 | 4.3 | 25.4 | 24.5 | 21. 6 | 16.9 | 14.9 |
| 10 | 3. 5 | 3. 1 | 2. 3 | 1. 5 | 1. 3 | 8.0 | 7.6 | 7.1 | 5.6 | 4.2 | 25.1 | 24.1 | 21. 6 | 17.2 | 14.7 |
| Measur ed average value | 3. 6 | 3. 0 | 2. 3 | 1. 4 | 1. 2 | 8.2 | 7.6 | 6.8 | 5.5 | 4.6 | 26.0 | 23.2 | 21. 1 | 16.9 | 14.9 |
| Absolut e deviati on from 40% HCT blood sample | 1. 25 | 0. 67 | / | 0. 89 | 1. 13 | | | | | | | | | | |
| Relativ e deviati on from 40% HCT blood sample | | | | | | 20.2 1% | 11.6 5% | / | 18.4 4% | 32.4 5% | 23.6 0% | 10.2 6% | / | 19.9 4% | 29.4 4% |
| Absolut e deviati on from the bioche mical analyze r | 1. 59 | 1. 11 | 0. 53 | - 0. 04 | - 0. 26 | | | | | | | | | | |
| Relativ e deviati | | | | | | 26.6 5% | 19.3 1% | 2.4 2% | - 11.9 4% | - 28.1 0% | 28.9 3% | 15.8 1% | 4.5 4% | - 15.9 7% | - 25.4 6% |
| on from the bioche mical analyze r | | | | | | | | | | | | | | | |

**Table 21 Verification report on uric acid concentration accuracy**

| Blood sample | 1 | | | | | 2 | | | | | 3 | | | | |
|---|---|---|---|---|---|---|---|---|---|---|---|---|---|---|---|
| Bioche mical value (mmol/ L) | 232 | 23 0 | 23 5 | 23 6 | 24 1 | 51 9 | 522 | 52 3 | 528 | 527 | 849 | 84 5 | 850 | 855 | 857 |
| Bioche mical mean | 232 | 23 3 | 23 9 | 24 1 | 24 5 | 52 2 | 520 | 53 2 | 523 | 525 | 844 | 84 7 | 852 | 853 | 859 |
| Blood HCT (%) | 10 % | 25 % | 42 % | 55 % | 70 % | 10 % | 25 % | 42 % | 55% | 70% | 10 % | 25 % | 42 % | 55% | 70% |

| Serial number | Uric acid concentration value (umol/L) | | | | | | | | | | | | | | |
|---|---|---|---|---|---|---|---|---|---|---|---|---|---|---|---|
| 1 | 387 | 29 8 | 24 3 | 19 9 | L O | 670 | 600 | 53 3 | 472 | 412 | 101 0 | 89 9 | 846 | 776 | 698 |
| 2 | 389 | 30 0 | 24 6 | 18 7 | L O | 682 | 603 | 54 4 | 477 | 400 | 977 | 91 0 | 850 | 770 | 687 |
| 3 | 375 | 31 0 | 24 6 | 18 5 | L O | 683 | 612 | 54 2 | 469 | 417 | 986 | 91 2 | 851 | 768 | 682 |
| 4 | 356 | 31 2 | 25 2 | 18 6 | L O | 679 | 623 | 55 0 | 459 | 400 | 988 | 91 3 | 855 | 762 | 681 |
| 5 | 368 | 31 2 | 24 5 | 19 1 | L O | 688 | 615 | 54 9 | 467 | 399 | 102 0 | 91 7 | 854 | 761 | 667 |
| 6 | 369 | 31 3 | 23 8 | 18 7 | L O | 687 | 617 | 53 9 | 462 | 389 | 987 | 90 2 | 849 | 755 | 668 |
| 7 | 367 | 30 5 | 24 2 | 18 2 | L O | 669 | 619 | 55 1 | 463 | 395 | 991 | 92 3 | 851 | 769 | 677 |
| 8 | 359 | 30 6 | 24 5 | 18 2 | L O | 683 | 622 | 55 5 | 459 | 396 | 993 | 90 5 | 855 | 777 | 671 |
| 9 | 357 | 31 6 | 24 5 | 18 0 | L O | 681 | 621 | 54 7 | 467 | 402 | 991 | 91 2 | 844 | 752 | 670 |
| 10 | 359 | 32 1 | 24 3 | 18 0 | L O | 680 | 623 | 54 6 | 455 | 391 | 998 | 91 7 | 846 | 751 | 680 |
| Measur ed average value | 368 .6 | 30 9.3 | 24 4.5 | 18 5.9 | / | 680. 2 | 615. 5 | 54 5.6 | 465. 0 | 400. 1 | 994. 1 | 91 1.0 | 850 .1 | 764. 1 | 678. 1 |
| Absolut e deviati on from 40% HCT blood sample | 125 .10 | 65. 80 | / | - 57. 60 | / | | | | | | | | | | |
| Relativ e deviati on from 40% HCT blood sample | | | | | | 24.4 0% | 12.5 6% | / | - 14.9 6% | - 26.8 3% | 16.9 0% | 7.1 3% | / | - 10.1 5% | - 20.2 6% |
| Absolut e deviati on from the bioche mical analyze r | 136 .60 | 77. 80 | 7.5 0 | 52. 60 | / | | | | | | | | | | |
| Relativ e deviati on from the bioche mical analyze r | | | | | | 30.6 8% | 18.1 4% | 3.4 3% | 11.5 1% | 23.9 4% | 17.4 4% | 7.6 8% | 0.1 1% | 10.5 3% | 20.9 7% |

The accuracy verification results in Comparative Example 1 show that the test results of different HCT blood samples differ significantly from those of 40% HCT blood samples (the lower the hematocrit, the higher the test result; the higher the hematocrit, the lower the test result), and the test results of the blood samples at low and high hematocrit levels also differ significantly from those from the biochemical analyzer, further demonstrating that the hematocrit of blood has a significant impact on the measurement results.

In summary, the measurement method and system in Examples 1 and 2 of the present invention can calibrate the test results of different HCT blood samples. Different from the prior art, the measurement method in Example 1 of the present invention can simultaneously implement the measurement of two indexes and HCT correction, and the measurement results do not cause relevant interference, where the CV of blood glucose concentration can be controlled within 4.0%; and the CV of uric acid concentration can be controlled within 4.5%.

### Comparative Example 2

This example employed test strips identical to Example 7, employed a single frequency for hematocrit correction, and employed the accuracy verification method in Example 7 to evaluate the accuracy of the test strips. The test results are shown in Table 22.

**Table 22 Verification report on cholesterol concentration accuracy**

| Blood sample | 1 | | | | | 2 | | | | | 3 | | | | |
|---|---|---|---|---|---|---|---|---|---|---|---|---|---|---|---|
| Biochemical value (mmol/L) | 2. 83 | 2.7 8 | 2. 84 | 2.7 6 | 2.6 9 | 5.2 3 | 5.16 | 5.2 0 | 5.1 8 | 5.2 2 | 7.2 0 | 7.2 2 | 7.2 4 | 7.23 | 7.2 2 |
| | 2. 80 | 2.7 9 | 2. 81 | 2.7 4 | 2.6 8 | 5.2 6 | 5.12 | 5.2 1 | 5.2 0 | 5.2 0 | 7.2 3 | 7.2 0 | 7.2 3 | 7.18 | 7.1 9 |
| Biochemical mean | 2. 82 | 2.7 9 | 2. 83 | 2.7 5 | 2.6 9 | 5.2 5 | 5.14 | 5.2 1 | 5.1 9 | 5.2 1 | 7.2 2 | 7.2 1 | 7.2 4 | 7.21 | 7.2 1 |
| Blood HCT (%) | 0 % | 20 % | 42 % | 55 % | 70 % | 0% | 20 % | 42 % | 55 % | 70 % | 0% | 20 % | 42 % | 55 % | 70 % |

| Serial number | Concentration value (mmol/L) | | | | | | | | | | | | | | |
|---|---|---|---|---|---|---|---|---|---|---|---|---|---|---|---|
| 1 | 3. 8 | 3.7 | 3. 0 | 2.6 | 2.1 | 7.1 | 6.0 | 5.6 | 5.6 | 4.9 | 8.6 | 8.2 | 7.4 | 6.8 | 6.4 |
| 2 | 3. 5 | 2.6 | 2. 7 | 2.6 | 2.1 | 6.8 | 6.0 | 5.6 | 5.3 | 4.7 | 8.8 | 8.1 | 7.6 | 6.9 | 6.5 |
| 3 | 3. 6 | 2.7 | 2. 8 | 2.6 | 2.1 | 6.8 | 6.1 | 5.4 | 5.3 | 4.8 | 8.7 | 8.0 | 7.7 | Outl ier | 6.4 |
| 4 | 3. 7 | 2.5 | 2. 9 | 2.4 | 2.2 | 6.9 | 5.8 | 5.3 | 5.8 | 4.9 | 8.6 | 8.2 | 7.6 | 6.8 | 6.3 |
| 5 | 3. 6 | 2.6 | 2. 9 | 2.6 | 2.0 | 6.8 | Outl ier | 5.3 | 5.4 | 5.0 | 8.9 | 8.0 | 7.3 | 7.0 | 6.7 |
| 6 | 3. 9 | 2.7 | 2. 8 | 2.5 | 1.9 | 7.0 | 5.9 | 5.4 | 5.8 | 4.6 | 8.6 | 7.8 | 7.5 | 6.9 | 6.4 |
| 7 | 4. 1 | 2.7 | 2. 7 | 2.6 | 2.0 | 6.5 | 5.8 | 5.5 | 5.2 | 4.8 | 8.9 | 7.9 | 7.7 | 7.0 | 6.4 |
| 8 | 3. 9 | 2.7 | 2. 6 | 2.5 | 2.1 | 6.8 | 6.2 | 5.3 | 5.5 | 5.1 | 8.9 | 8.2 | 7.4 | 6.8 | 6.4 |
| 9 | 4. 0 | 2.6 | 2. 8 | 2.4 | 1.9 | 6.9 | 5.8 | 5.5 | 5.5 | 5.1 | 8.5 | 8.0 | 7.4 | 6.8 | 6.6 |
| 10 | 3. 7 | 2.6 | 2. 8 | 2.5 | 2.0 | 6.9 | 6.2 | 5.6 | 5.5 | 4.9 | 8.5 | 8.3 | 7.7 | 7.0 | 6.7 |
| Measured average value | 3. 8 | 2.7 | 2. 8 | 2.5 | 2.0 | 6.8 | 6.0 | 5.4 | 5.5 | 4.9 | 8.7 | 8.1 | 7.5 | 6.9 | 6.5 |
| Absolute deviation from 40% HCT blood sample | 1. 00 | 0.0 6 | / | 0.2 7 | 0.7 7 | | | | | | | | | | |
| Relative deviation from | | | | | | 25. 9% | 11.1 % | / | 0.9 % | 10. 3% | 15. 2% | 6.9 % | / | 8.4 % | 14. 2% |
| 40% HCT blood sample | | | | | | | | | | | | | | | |
| Absolute deviation from the biochemical analyzer | 0. 98 | - 0.0 5 | - 0. 03 | - 0.2 2 | - 0.6 5 | | | | | | | | | | |
| Relative deviation from the biochemical analyzer | | | | | | 30. 6% | 17.6 % | 4.5 % | 5.8 % | - 6.4 % | 20. 4% | 11. 8% | 4.3 % | - 4.1 % | - 10. 1% |

In Comparative Example 2, the accuracy verification results show that, when the hematocrit is 0%, the relative deviation from the 40% HCT blood sample exceeds 15%, demonstrating inadequate HCT correction capability. This indirectly proves that, when a single frequency is used for impedance measurement, the hematocrit of blood samples cannot be 0%. In addition, during correction, two test values appeared as outliers, likely due to significant deviations in impedance values, which consequently led to excessive deviations in the corrected concentration values.

In summary, the measurement method in Example 3 and the measurement system in Example 4 of the present invention can calibrate the test results of blood samples with 0-70% HCT levels, where the test samples can be identical to biochemical analyzer test samples, allowing direct plasma measurement; and the relative deviations between the measured values and true values can be controlled within ±6.5%.

Although the preferred embodiments of the present application are described, those skilled in the art can make changes and modifications to these embodiments once they learn the basic inventive concept. Therefore, the appended claims are intended to be interpreted as including the preferred embodiments and all the changes and modifications falling into the scope of the present application.

Apparently, those skilled in the art can make various modifications and variations to the present application without departing from the spirit and scope of the present application. Provided that these modifications and variations of the present application fall into the scope of the claims of the present application and equivalent technologies thereof, the present application is intended to include these modifications and variations.

## Claims

1. A metabolic index concentration measurement method, comprising the following steps:
S1, measuring an impedance value and a metabolic index current value of a blood sample; comparing the measured impedance value with a preset standard impedance library, and determining a hematocrit range corresponding to the blood sample based on the comparison result, wherein the preset standard impedance library comprises different hematocrit levels and standard impedance values corresponding to the hematocrit levels;
S2, searching for metabolic index concentration equations corresponding to the hematocrit range;
S3, substituting the metabolic index current value into the metabolic index concentration equations Y1 and Y2 to obtain metabolic index concentrations C1 and C2 respectively; and
S4, substituting the metabolic index concentrations C1 and C2 into the following formula to calculate a metabolic index concentration value Fn: Fn={(a-a1)C2/(a2-a1)}+{(a2-a)C1/(a2-a1)};
wherein Y1 and Y2 represent the metabolic index concentration equations corresponding to an upper limit value and a lower limit value of the hematocrit range respectively, "a" in the above formula represents the measured impedance value of the blood sample, "a1" represents that the hematocrit value is a standard impedance value corresponding to the lower limit value of the hematocrit range, and "a2" represents that the hematocrit value is a standard impedance value corresponding to the upper limit value of the hematocrit range.

2. The metabolic index concentration measurement method according to claim 1, wherein in step S1, a process of obtaining standard impedance values in the standard impedance library comprises:
1) obtaining a plurality of original blood samples;
2) for each of the original blood samples, preparing M blood samples with hematocrit levels of N1%, N2%... NM%, wherein N1, N2... NM are all positive integers, and N1 < N2 <... < NM; and
3) measuring the impedance values of each blood sample at the corresponding hematocrit levels respectively, and determining an average value of all impedance values at the same hematocrit level as the standard impedance value corresponding to that hematocrit level.

3. The metabolic index concentration measurement method according to claim 1, wherein in step S1, if it is determined based on the comparison result that the hematocrit value of the blood sample is equal to the lower limit value or upper limit value of the hematocrit range, the lower limit value and upper limit value of the hematocrit range corresponding to the blood sample are set to be equal to the hematocrit value of the blood sample determined based on the comparison result; and
then step S4 is replaced as:
the metabolic index concentration value Fn=C1 or C2.

4. The metabolic index concentration measurement method according to claim 1, wherein in step S1, if it is determined based on the comparison result that the difference between the hematocrit value of the blood sample and a minimum value of lower limit values of all hematocrit ranges is not greater than a second set threshold, the lower limit value and upper limit value of the hematocrit range corresponding to the blood sample are both set as the minimum value; if it is determined based on the comparison result that the difference between the hematocrit value of the blood sample and a maximum value of upper limit values of all hematocrit ranges is not greater than the second set threshold, the lower limit value and upper limit value of the hematocrit range corresponding to the blood sample are both set as the maximum value; and
then step S4 is replaced as:
the metabolic index concentration value Fn=C1 or C2.

5. The metabolic index concentration measurement method according to claim 1, wherein in step S2, a process of establishing metabolic index concentration equations comprises: obtaining an original blood sample, preparing the original blood sample into M blood samples with different hematocrit levels, dividing the blood sample corresponding to each hematocrit level into M equal parts, preparing the M equal parts of blood samples into blood samples with different metabolic index concentrations, and calibrating the blood samples with different hematocrit levels and different metabolic index concentrations to establish a metabolic index concentration - current value relation curve, thereby obtaining the metabolic index concentration equations corresponding to different hematocrit levels.

6. The metabolic index concentration measurement method according to claim 5, wherein an expression of the blood glucose concentration - blood glucose current value relation curve is: Yi=b*xᵢ²+c*xᵢ+d, wherein xᵢ represents a blood glucose current value, Yᵢ represents a blood glucose concentration value, and b, c, and d all represent constants; and an expression of the uric acid concentration - uric acid current value relation curve is: Jᵢ=e*yᵢ+f, wherein e and f all represent constants, yᵢ represents a uric acid current value, and Jᵢ represents a uric acid concentration value.

7. A metabolic index concentration measurement method, comprising the following steps:
A. applying a plurality of different frequencies to an electrode module of an electrochemical test strip;
B. obtaining concentration values of a metabolic index of a blood sample at different frequencies; and
C. performing weighted averaging on the concentration values of the metabolic index of the same blood sample at the different frequencies to obtain a corrected concentration value of the metabolic index corresponding to the blood sample;
wherein the concentration value of the metabolic index of the blood sample at a certain frequency is obtained by the method according to one of claims 1 to 6.

8. The metabolic index concentration measurement method according to claim 7, wherein after step A and before step B, the method further comprises:
comparing the impedance values at different frequencies with a standard impedance library of different frequencies to determine whether the hematocrit ranges of the blood sample at different frequencies meet set requirements, and if so, performing step B; otherwise, issuing an alarm instruction.

9. The metabolic index concentration measurement method according to claim 8, wherein the set requirements comprise:
if the number of frequencies n is 2, the hematocrit ranges selected at the two frequencies are located within an adjacent or same hematocrit range;
if the number of frequencies n is 3, at least two of the hematocrit ranges selected at the three frequencies are located within a same hematocrit range, and the other one is located within an adjacent hematocrit range; and
if the number of frequencies n is greater than or equal to 4, at least n-2 of the hematocrit ranges selected at n different frequencies are located within a same hematocrit range, and the remaining ones are located within adjacent hematocrit ranges.

10. A metabolic index concentration measurement system, comprising:
an HCT measurement module, configured to measure an impedance value, compare the measured impedance value with a preset standard impedance library, determine a hematocrit range corresponding to the blood sample based on the comparison result, and search for metabolic index concentration equations corresponding to the hematocrit range; a metabolic index current value measurement module, configured to measure a metabolic index current value of the blood sample; and
a calculation unit, configured to substitute the metabolic index current value obtained by the metabolic index current value measurement module into metabolic index concentration equations Y1 and Y2 respectively to obtain metabolic index concentrations C1 and C2, and substitute the metabolic index concentrations C1 and C2 into the following formula to calculate a metabolic index concentration value Fn: Fn={(a-a1)C2/(a2-a1)}+{(a2-a)C1/(a2-a1)};
wherein Y1 and Y2 represent blood glucose concentration equations corresponding to an upper limit value and a lower limit value of the hematocrit range respectively, a represents the measured impedance value of the blood sample, a1 represents that the hematocrit value is a standard impedance value corresponding to the lower limit value of the hematocrit range, and a2 represents that the hematocrit value is a standard impedance value corresponding to the upper limit value of the hematocrit range.

11. The metabolic index concentration measurement system according to claim 10, wherein the standard impedance library and metabolic index concentration equations corresponding to each hematocrit range are all stored in a data unit.

12. The metabolic index concentration measurement system according to claim 10 or 11, wherein a process of obtaining standard impedance values in the standard impedance library comprises:
obtaining a plurality of original blood samples;
for each of the original blood samples, preparing M blood samples with hematocrit levels of N1%, N2%... NM%, wherein N1, N2... NM are all positive integers, and N1 < N2 <... < NM; and
measuring the impedance values of each blood sample at the corresponding hematocrit levels respectively, and determining an average value of all impedance values at the same hematocrit level as the standard impedance value corresponding to that hematocrit level.

13. The metabolic index concentration measurement system according to claim 12, wherein an interval between two adjacent hematocrit values is 10%.

14. The metabolic index concentration measurement system according to claim 10 or 11, wherein a process of establishing metabolic index concentration equations comprises: obtaining an original blood sample, preparing the original blood sample into M blood samples with different hematocrit levels, dividing the blood sample corresponding to each hematocrit level into M equal parts, preparing the M equal parts of blood samples into blood samples with different metabolic index concentrations, and calibrating the blood samples with different hematocrit levels and different metabolic index concentrations to establish a metabolic index concentration - current value relation curve, thereby obtaining the metabolic index concentration equations corresponding to different hematocrit levels.

15. The metabolic index concentration measurement system according to claim 10, further comprising:
a display unit, configured to display the metabolic index concentration value Fn.

16. A metabolic index concentration measurement system, comprising:
a frequency control unit, configured to apply a plurality of different frequencies to an electrode module of an electrochemical test strip;
a metabolic index concentration value obtaining unit, configured to obtain concentration values of a metabolic index of a blood sample at different frequencies; and
a corrected concentration value calculation unit, configured to perform weighted averaging on the concentration values of the metabolic index of the same blood sample at the different frequencies to obtain a corrected concentration value of the metabolic index corresponding to the blood sample;
wherein the metabolic index concentration value obtaining unit is the metabolic index concentration measurement system according to one of claims 10 to 15.

17. The metabolic index concentration measurement system according to claim 16, further comprising a determination unit, configured to compare the impedance values at different frequencies with a standard impedance library of different frequencies to determine whether the hematocrit ranges of the blood sample at different frequencies meet set requirements, and if so, send a working instruction to the metabolic index concentration value obtaining unit, otherwise, issue an alarm instruction.

18. The metabolic index concentration measurement system according to claim 17, wherein the set requirements comprise:
if the number of frequencies n is 2, the hematocrit ranges selected at the two frequencies are located within an adjacent or same hematocrit range;
if the number of frequencies n is 3, at least two of the hematocrit ranges selected at the three frequencies are located within a same hematocrit range, and the other one is located within an adjacent hematocrit range; and
if the number of frequencies n is greater than or equal to 4, at least n-2 of the hematocrit ranges selected at n different frequencies are located within a same hematocrit range, and the remaining ones are located within adjacent hematocrit ranges.

19. An electrochemical measurement system, comprising:
an electrochemical test strip; and
a linker, configured to transmit corresponding reaction signals generated by a sample on the electrochemical test strip to a measurement unit;
wherein the measurement unit is the metabolic index concentration measurement system according to one of claims 7 to 18.
